# EUROPEAN PATENT APPLICATION

(11) **EP 2 219 029 A1**
(43) Date of publication of application: **18.08.2010**
(21) Application number: 09290067.9
(22) Date of filing: 30.01.2009
(51) Int. Cl.: G01N 33/50, C12N 5/07

(54) **Test systems, methods and uses involving AS160 protein**

(71) Applicant: Sanofi-Aventis, 75013 Paris (FR)
(72) Inventor: The designation of the inventor has not yet been filed
(74) Representative: Körner, Kathrin

(57) **Abstract**

The present invention relates to a method of identifying a substance altering glucose uptake and/or GLUT4 translocation to the plasma membrane of a cell comprising contacting a test system comprising AKT substrate 160kDa- protein (AS160- protein) with a test substance, and identifying a test substance as a substance altering glucose uptake of a cell by detecting a signal indicative for altered glucose uptake of a cell; a test system comprising a gene coding for the AKT substrate 160 kDa- protein (AS160- protein) and an inducible promoter providing for controllable expression of the gene; the use of the test system for the identification of a substance improving glucose uptake and/or GLUT4 translocation to the plasma membrane of a cell; and the use of AS160- protein in a model for type 2 diabetes.

## Description

The present invention relates to a method of identifying a substance altering glucose uptake of a cell comprising contacting a test system comprising AKT substrate 160kDA protein (AS 160) with a test substance, and identifying a test substance as a substance altering glucose uptake of a cell by detecting a signal indicative for altered glucose uptake of a cell; a test system comprising a gene coding for AKT substrate 160kDA protein (AS 160) and an inducible promoter providing for controllable expression of the gene; the use AKT substrate 160kDA protein (AS 160) and of the test system for the identification of a substance improving glucose uptake into a cell; and the use of AKT substrate 160kDA protein (AS 160) in a model for type 2 diabetes.

Diabetes mellitus is a metabolic disorder characterized by hyperglycemia and other signs, as distinct from a single illness or condition. The World Health Organization recognizes three main forms of diabetes: type 1, type 2, and gestational diabetes (occurring during pregnancy), which have similar signs, symptoms, and consequences, but different causes and population distributions. Ultimately, all forms are due to the beta cells of the pancreas being unable to produce sufficient insulin to prevent hyperglycemia.

Type 1 is usually due to autoimmune destruction of the pancreatic beta cells which produce insulin. Type 2 is characterized by tissue-wide insulin resistance, particularly of insulin-sensitive tissues comprising adipose tissue, liver and skeletal muscle, and varies widely; it sometimes progresses to loss of beta cell function. Gestational diabetes is similar to type 2-diabetes, in that it involves insulin resistance caused by hormones of pregnancy.

Types 1 and 2 are incurable chronic conditions, but have been treatable and are usually managed with a combination of dietary treatment and medicaments including insulin supplementation.

Diabetes can cause many complications such as hypoglycemia, ketoacidosis or nonketotic hyperosmolar coma. Serious long-term complications include cardiovascular disease (doubled risk), chronic renal failure (diabetic nephropathy is the main cause of dialysis in developed world adults), retinal damage (which can lead to blindness and is the most significant cause of adult blindness in the non-elderly in the developed world), nerve damage (of several kinds), and microvascular damage, which may cause erectile dysfunction (impotence) and poor healing. Poor healing of wounds, particularly of the feet, can lead to gangrene which can require amputation - the leading cause of non-traumatic amputation in adults in the developed world.

Because insulin is the principal hormone that regulates uptake of glucose into most cells from the blood (primarily muscle and adipocytes), deficiency of insulin or the insensitivity of its receptors plays a central role in all forms of diabetes mellitus. Insulin is released into the blood by β- cells in the pancreas in response to rising levels of blood glucose (e.g., after a meal). Insulin enables most body cells (about 2/3 is the usual estimate, including muscle cells and adipose tissue) to absorb glucose from the blood.

Type 2 diabetes mellitus is due to a combination of defective insulin secretion and insulin resistance or reduced insulin sensitivity of insulin-sensitive tissues, particularly adipose tissue, liver and skeletal muscle. In the early stage the predominant abnormality is reduced insulin sensitivity, characterized by elevated levels of insulin in the blood. At this stage, hyperglycemia can be reversed by a variety of measures and medications that improve insulin sensitivity or reduce glucose production by the liver, but as the disease progresses, the impairment of insulin secretion worsens, and therapeutic replacement of insulin often becomes necessary.

Usually, type 2 diabetes is first treated by attempts to change physical activity, the diet (generally to decrease carbohydrate intake), and weight loss. The usual next step, if necessary, is treatment with oral antidiabetic drugs. As insulin production is initially only moderately impaired in type 2 diabetics, oral medication can still be used to improve insulin production, to regulate inappropriate release of glucose by the liver and to substantially attenuate insulin resistance.

Adequate treatment of diabetes in early the stage, particularly improvement insulin sensitivity of adipose tissue, liver and skeletal muscle, may protract, retard and/or prevent progression of the disease.

Accordingly, a first object of the invention was to better understand the molecular background involved in the glucose metabolism, thus helping to better search for new potential drugs improving insulin sensitivity of cells, particularly of cells of skeletal muscle, adipose tissue and/or liver, which are the main insulin-sensitive tissues.

AS160 (AKT substrate 160 kDa) also known as TBC1D4, is phosphorylated in response to insulin by AKT in adipocytes and skeletal muscle cells. Most interestingly, activation of AS160 is reduced in diabetic patients accompanied with impaired GLUT4 translocation. Therefore, AS160 may provide a link between the AKT signalling cascade and the translocation of GLUT4 after stimulation with insulin.
The role of full-length AS160 in adipocytes and skeletal muscle cells is already examined and described. According to the literature, overexpression of full-length AS160 does not alter the basal or insulin-stimulated surface-to-total distribution of GLUT4, indicating that the amount of AS160 seems not to be rate-limiting {Zeigerer, 2004 21 /id; Sano, 2003 7 /id}. To investigate the function of AS160 protein in muscle cells a rat skeletal muscle cell line stably and inducibly expressing AS160 was established. Overexpression of AS160 induced a significant increase of 2-deoxyglucose uptake after stimulation with insulin. Insulin-mediated 2-deoxyglucose uptake was nearly completely abrogated with Wortmannin, indicating that AKT is involved in the activation of AS160 protein. Taken together, these data provide the basis for the development of a highly sensitive cell-based high throughput screening assay.

Insulin induces the uptake of glucose into fat and muscle cells. Binding of insulin to its receptor (IR) leads to activation of phosphatidylinositol-3-kinase (PI3K) and subsequently to the activation of AKT (PKB, protein kinase B) as well as downstream effectors of AKT. A key event in this process is the translocation of the GLUT4/SLC2A4 to the plasma membrane, which actively regulates the uptake of glucose into the cells {Watson, 2006 9 /id}. This trafficking process is in part mediated by AS160.
AS160 (TBC1 D4) was recently identified as an AKT substrate that is critically involved in insulin-stimulated regulation of GLUT4 trafficking in 3T3L1 adipocytes {Kane, 2002 10 /id}. Additional studies demonstrated that AS160 also plays a role in skeletal muscles of mice, rats and humans {Bruss, 2005 15 /id; Deshmukh, 2006 14 /id;Treebak, 2006 13 /id}. Insulin, contraction or AICAR (5-aminoimidozole-4-carboxamide 1 β-ribonucleoside, AMPK activator) increase phosphorylation of AS160 on two sites (Ser 588 and Thr 642) which lie in characteristic motifs predicted for AKT phosphorylation (RXRXXS/T) {Kane, 2002 10 /id}. Interestingly, AS160 activation is reduced in patients with type 2 diabetes, which is accompanied with impaired GLUT4 translocation {Karisson, 2005 11 /id}. Published data reporting the overexpression of full-length AS160 in adipocytes did not alter the basal or insulin-stimulated surface-to-total distribution of GLUT4, indicating that the amount of AS160 seems not to be rate-limiting {Zeigerer, 2004 21 /id;Sano, 2003 7 /id}. Experiments with mutant AS160, containing 4 mutated phosphorylation sites, showed that GLUT4 translocation is markedly reduced {Sano, 2003 7 /id}. A prominent feature of AS160 is the presence of a GTPase activating domain for Rab proteins. These small G-proteins are required for membrane trafficking. A functional GAP domain of AS160 is required for GLUT4 translocation {Eguez, 2005 8 /id}. Meanwhile there is considerable evidence that in the absence of insulin the GAP domain of AS160 keeps a critical Rab protein in its inactive GDP-bound state. Upon stimulation with insulin AS160 is phosphorylated which in turn suppresses its GAP activity and leads to the conversion of Rab-GDP into its active GTP bound version and thereby allows trafficking of GLUT4 containing vesicles to and docking with the plasma membrane. The question whether or not AS160 is directly associated with GLUT4 vesicles is still controversial {Kane, 2002 10 /id;Kane, 2002 10 /id;Larance, 2005 20 /id}. Additionally, the contribution of a variety of Rab proteins to AS160 mediated effects is currently examined in adipocytes and muscle cells {Ishikura, 2007 140 /id}.

Besides, recently a closely related protein called TBC1D1 was identified in the mouse genome {Ishikura, 2007 140 /id;Roach, 2007 145 /id}. This protein is also a likely AKT substrate and seems to be involved in the GLUT4 translocation process. However the mode of molecular interaction between AKT and GLUT4 vesicles remained unknown.

Surprisingly, and in contrast to the published data obtained using adipocytes and muscle cells, studies of the inventors showed that overexpression of full-length AS160 in a rat myoblast cell line co-expressing GLUT4myc, significantly enhances insulin-stimulated glucose uptake rates.

As an ineffective insulin action is a hallmark of type 2 diabetes, AS160 provides a novel target to study the molecular basis of insulin-resistance in insulin-sensitive tissues. Additionally, AS160 may be used to identify substances which could improve glucose uptake, and therefore insulin sensitivity, in the relevant tissues.

They also found that a test system involving AS160 may be used to study glucose uptake of cells under high glucose conditions. This may be particularly important for a diabetes model or for the identification of a suitable therapeutic for the treatment and/or prevention of diabetes, as this disease is characterized by increased levels of glucose in the blood. Accordingly, testing a substance capable of altering, particularly improving, glucose uptake under this condition (high glucose) might be beneficial for the identification of a new therapeutic.

Accordingly, AS160 may be used in a method of identifying a substance altering, particularly increasing, glucose uptake of a cell and, therefore, having a potential for the treatment or prevention of type 2 diabetes.

Therefore, the present invention provides in a first aspect a method of identifying a substance altering glucose uptake and/or GLUT4 translocation of a cell comprising
(a) contacting a test system comprising AKT substrate 160kDa protein (AS160-protein) with a test substance, and
(b) identifying a test substance as a substance altering glucose uptake and/or GLUT4 translocation of a cell by detecting a signal indicative for altered glucose uptake of a cell.

"Altering glucose uptake of a cell" in the context of the present invention means a change, either increase or decrease, of glucose uptake of a cell. Preferably the glucose uptake of a cell is increased.

In the context of the present invention, the glucose uptake of a cell is altered, i.e. decreased or preferably increased, in comparison to a control, if the glucose uptake of a cell contacted with the (test) substance is significantly lower or higher, respectively, than that of a control (e.g. the same cell not contacted with the (test) substance). The person skilled in the art knows statistical procedures to assess whether two values are significantly different from each other such as Student's t-test or chi-squared test (see also Examples for suitable test methods).

In a preferred embodiment of increased glucose uptake, the glucose uptake of a cell amounts to at least 110 %, preferably to at least 125 %, more preferably to at least 150 %, 160 %, 170 %, 180 % or 190 %, still more preferably to at least 200 % and most preferably to at least 300 % of the control.

As detailed above, for the treatment or prevention of type 2 diabetes it is particularly important to modify glucose uptake of an insulin-sensitive tissue. Accordingly, it is preferred that the method of the invention allows for the identification of a substance altering, preferably increasing, the glucose uptake in at least one, preferably at least two, more preferably at least three or at least four or at least five or at least six insulin-sensitive tissue(s). Examples of insulin-sensitive tissues include, without limitation, adipose tissue, liver, skeletal muscle, pancreatic tissue, myocardium, vascular smooth muscle and active mammary gland.

However, the six main insulin-sensitive tissues are adipose tissue, liver skeletal muscle, heart, brain and pancreatic tissue. Accordingly, the substance preferably alters, more preferably increases, glucose uptake in at least one, two, three, four, five or six or more of these tissues, i.e. adipose tissue, skeletal muscle, heart, brain, pancreatic tissue and/or liver.

If glucose uptake is altered, preferably increased, in one tissue, this could be, for example, any of: adipose tissue, liver, heart, brain, pancreatic tissue or skeletal muscle.

If glucose uptake is altered, preferably increased, in two tissues, this could be, for example,
adipose tissue and liver;
adipose tissue and skeletal muscle; or
liver and skeletal muscle, or any other combination of two of the six main insulin-sensitive tissues as listed above.

If glucose uptake is altered, preferably increased, in three tissues, this could be, for example, adipose tissue, liver and skeletal muscle or any other combination of three of the above-listed six main insulin-sensitive tissues.

If glucose uptake is altered, preferably increased in four tissues, this could be, for example, adipose tissue, liver, skeletal muscle and brain or any other combination of four of the above-listed six main insulin-sensitive tissues.

If glucose uptake is altered, preferably increased in five tissues, this could be, for example, adipose tissue, liver, skeletal muscle, brain and heart, or any other combination of five of the above-listed six main insulin-sensitive tissues.

The main cell types present in adipose tissue, liver, heart, brain, pancreatic tissue and skeletal muscle are adipocytes, hepatocytes, heart muscle cells, neuronal cells pancreatic cells, beta cells and skeletal muscle cells, respectively. Accordingly, the substance preferably alters, more preferably increases, glucose uptake in at least one, two or three of these cell types, i.e. adipocytes, hepatocytes and/or skeletal muscle cells.

If glucose uptake is altered, preferably increased, in one cell type, this could be, for example in adipocytes, hepatocytes, heart muscle cells, neuronal cells pancreatic cells, beta cells or skeletal muscle cells.
If glucose uptake is altered, preferably increased, in two cell types, this could be, for example,
adipocytes and hepatocytes;
adipocytes and skeletal muscle cells; or
hepatocytes and skeletal muscle cells or any other combination of the main cell types present in the six main insulin-sensitive tissues as listed above.

If glucose uptake is altered, preferably increased, in three cell types, this could be, for example, adipocytes, hepatocytes and skeletal muscle cells or any other combination of the above listed main cell types present in the six main insulin-sensitive tissues as listed above.

If glucose uptake is altered, preferably increased in four, five or more of the main cell types as listed above for the six main insulin-sensitive tissues, this can be any combination of four, five or more of those cell types.

As detailed above, the method of the invention involves a test system comprising AS160- protein.
Additionally, mutations within the protein may be present as detailed below.

AS160 (AKT substrate 160kDa; NM_014832 (NCBI)) was originally identified as a substrate of the protein kinase AKT in 3T3 adipocytes (Kane et al., 2002). If, in the context of this application, it is referred to AS160 or AS 160 full length, the isoform 1 is meant, as discussed in the above-cited publications. If recently-identified isoforms 2 or 3 (lacking exon 11 (isoform 2) or 11 and 12 (isoform 3) of AS 160 isoform1) are meant, they are always identified as either isoform 2 or isoform 3 (see below),

The sequence of the human AS160 gene (gene ID: 9882) is known in the art and can be retrieved under NC_000013.9 at the NCBI Database. The human AS160 gene is located on chromosome 13, 13q22.2. The coding nucleic acid sequence of said gene can be retrieved from the NCBI database under the number NM_014832 (SEQ ID NO.1). The derived human protein sequence can be retrieved under the number NP_055647.2. The above sequences can be retrieved from the NCBI Database. NCBI is the National Center for Biotechnology Information (postal address: National Center for Biotechnology Information, National Library of Medicine, Bethesda, MD 20894, USA; web address: http://www.ncbi.nlm.nih.gov/).

In contrast to AS 160 as meant herein, in the gene encoding novel isoform 2 of AS160, exons 11 and 12 are missing in comparison to the full-length AS160 gene. In case of the human gene, the nucleotides encoding amino acids 678 to 740 are missing in comparison to the human full-length AS160 as defined by the sequence NM_014832 (see NCBI data base). Furthermore, two mismatches were identified at positions nt 606 (silent) and nt 3827 (Ala → Val). In addition, one clone contained a 3 bp deletion (nt 2594-2596) that was also found in human placenta cDNA but not in human brain cDNA. For the expression clone of the isoform 2 lacking exons 11 and 12, which was used, the deleted 3bp sequence was reintroduced to resemble more closely the full length sequence of NM_014832 (NCBI).

In contrast to AS 160 as meant herein, in the gene encoding isoform 3 of AS160, exon 12 is deleted compared to theAS160 gene. This exon corresponds to amino acids 733 to 740 with respect to NM_014832 (see NCBI database).

Studies demonstrated that AS160 also plays a role in skeletal muscles of mice, rats and humans. Insulin, contraction or AICAR (5-aminoimidozole-4-carboxamide 1 β-D-ribonucleoside, cAMP-dependent protein kinase (cAMPK) activator) increase phosphorylation of AS160 on two sites (Ser 588 and Thr 642) which lie in characteristic motifs predicted for AKT phosphorylation (RXRXXS/T) (Kane et al., supra). A prominent feature of AS160 is the presence of a GTPase activating domain for Rab proteins. These small G-proteins are required for membrane trafficking. In this context, recent data provide evidence that AS160 links signals downstream of AKT with the insulin-stimulated translocation of GLUT4 (Sano et al., 2003). AS160 activation is reduced in patients with type II diabetes, resulting in an impaired GLUT4 translocation (Karisson et al., 2005b). Overexpression of full-length AS160 in adipocytes did not alter the basal or insulin-stimulated surface-to-total distribution of GLUT4 indicating that the amount of AS960 seems not to be rate-limiting (Zeigerer et al., 2004; Sano et al., 2003). Experiments with mutant AS160 (containing 4 mutated phosphorylation sites) showed that GLUT4 translocation is markedly reduced (Sano et al., 2003). Additionally, a functional GAP (GTPase-activating protein) domain of AS160 is required for GLUT4 translocation.

Short mismatches with respect to the sequence according to SEQ ID No.2 may be present in the AS 160- protein. A short mismatch is intended to relate to an addition, deletion, or substitution of up to 5 adjacent amino acids, preferably up to 4, more preferably up to 3, still more preferably up to 2, most preferably up to 1 adjacent amino acid. Within the naturally occurring AS160- protein there may by up to 5 additions, deletions, and/or substitutions, preferably up to 4, more preferably up to 3, 2 or 1 additions, deletions, and/or substitutions in comparison to SEQ ID NO.2.

Exemplary deletions and substitutions are those mentioned above, namely a deletion of 1 amino acid at a position corresponding to that encoded by nt 2594-2596 of the human AS150 gene or a substitution (e.g. Ala → Val) at a position corresponding to that encoded by nt 3827-3829 of the human AS160 gene. It is noted that it is intend that the AS160- protein or the nucleic acid coding the same may also be derived from species other than human including, but not limited to mammal, such as monkey, rodent (e.g. mouse or rat), dog, cat, cattle, pig, horse, sheep, goat or to avian, such as chicken or to amphibian, such as frog; however, the mammalian or human amino acid and nucleic acid sequences are preferred. The positions in AS160 or AS160-like protein sequences of species other than human corresponding to positions of the human sequences specified herein may be determined by sequence alignments as known to the skilled practitioner.

In one embodiment of the invention AS 160- protein may comprise or consist of the sequence of a naturally occurring AS160- protein (such as SEQ ID NO: 2) and C-and/or N-terminal additions, such as short C- and/or N-terminal sequences of at most 30, 25, 20, 15, 10, 9, 8, 7, 6, 5, 4, 3, 2, or 1 amino acids heterologous to the protein as defined below.

Accordingly, the feature "AS160- protein" relates to e.g.:
1) a protein encoded by a nucleic acid comprising or having 90% or more, preferably 95% or more, more preferably 97% or more, more preferably 99% or more sequence homology with a nucleic acid sequence according to SEQ ID NO:1.
2) a protein encoded by a nucleic acid comprising or having the nucleic acid sequence according to SEQ ID NO:1,or
3) a protein encoded by a nucleic acid hybridizing with a nucleic acid having the nucleic acid sequence according to SEQ ID NO:1 under conditions of stringency, or
4) a protein having the amino acid sequence according to SEQ ID NO:2 or
5) a protein having an amino acid sequence of 95% or more, preferably 97% or more, more preferably 98% or more, more preferably 99% or more and preferably 99,5% or more sequence homology with SEQ ID NO:2,
8) a functional fragment or a functional derivative of one of the AS 160- protein as defined above under 1 to 7,, the above proteins preferably having at least one of the functional characteristics of AS 160- protein as specified above and below.

A fragment is a protein that carries one or more end-terminal (n- and/or c-terminal) or internal deletions of one, two or more amino acids, when compared to the full-length protein. A functional fragment of a protein is any fragment of this protein having at least one and preferably two or more of the functional characteristics of the full-length protein.

The term derivative of a protein comprises any type of modification of the protein in comparison to the naturally-occurring form (in the context of present application especially in comparison to AS 160 according to SEQ ID NO:2), that is not a deletion. A functional derivative of a protein is any derivative of this protein having at least one and preferably two or more of the functional characteristics of the unmodified protein.

Present invention also comprises functional derivatives of fragments of AS160-protein.

The determination of homology of amino acid or nucleic acid sequences can e.g. be made by use of the program GAP (GCG Program Package, Genetic Computer Group 1991) or any other of the programs known in the art.
Isolated polynucleotides and oligonucleotides can be used for hybridizing at different conditions of stringency.

A nucleic acid molecule can hybridise to another nucleic acid molecule when the single stranded forms of both molecules can anneal under suitable reaction ("annealing" or hybridisation) conditions (depending on temperature and ionic strength of the surrounding medium) to form a new double stranded nucleic acid molecule. Hybridisation requires that the two annealing nucleic acid molecules comprise complementary sequences. Depending on the selected annealing conditions, the stringency conditions, mismatches between the bases are possible without preventing double strand formation.

The term stringency describes reaction conditions that influence the specificity of hybridisation or annealing of two single stranded nucleic acid molecules. Stringency, and thus specificity of a reaction depends, inter alia, of the temperature and buffer-conditions used for a reaction: Stringency, and thus specificity, can e.g. be increased by increasing the reaction temperature and/or lowering the ion strength of the reaction-buffer. Suitable conditions of stringency for the hybridisation of nucleic acids depend on the length, the type of nucleic acid and their degree of complementarity. The variables are known in the state of the art. The greater the degree of similarity or homology between two annealing nucleotides sequences, the greater the melting temperature for hybridisation products of nucleic acids with those sequences. The relative stability of nucleic acid hybridisation is dependent according to the type of the single stranded nucleic acids forming the double strand: RNA:RNA>DNA:RNA>DNA:DNA. For hybridisation products of greater than 100 nucleotides in length, equations for calculating the melting temperature are known in the art. For shorter hybridisation products (e.g. oligonucleotides) the calculation of the melting temperature is dependent on the length, wherein mismatches become more important.

Conditions of low stringency (and thus low reaction and hybridisation specificity) exist for example, if a hybridisation is performed at room temperature in 2xSSC-solution. Conditions of high stringency comprise e.g. a hybridisation reaction at 68°C in 0,1×SSC and 0,1% SDS solution.

In the context of present invention the term "hybridising under conditions of stringency" refers to conditions for the performance of the hybridisation reaction and the following washing procedure, at which nucleotide sequences with a certain complementarity typically remain hybridised. The choice of such conditions for a given set of nucleic acids lies within the skill of the average artisan, and suitable protocols can be found in well known literature for standard methods like, for example, "Current Protocols in Molecular Biology", John Wiley & Sons, N.Y. (1989), 6.3.1 to 6.3.6.

Hybridisation under conditions of stringency within the different aspects of present invention is preferably understood to be:
Hybridising a labelled probe with a nucleic acid sample to be analysed at 65°C, or in the case of oligonucleotide probes, at 5°C below the annealing or melting temperature of the duplex consisting of oligonucleotide and sample (annealing and melting temperature are in the following understood to be synonyms) over night in 50mM Tris pH 7,5, 1M NaCl, 1% SDS, 10% Dextran Sulfate, 0,5 mg/ml denatured salmon or herring sperm DNA.
Washing for 10 minutes in 2xSSC at room temperature.
Washing for 30 minutes in 1xSSC/0,1%SDS at 65°C (or in the case of oligonucleotides: 5°C below the annealing temperature).
Washing for 30 minutes in 0,1xSSC10,1%SDS at 65°C (or in the case of oligonucleotides: 5°C below the annealing temperature).

In one embodiment, the AS160- protein is encoded by a nucleic acid comprising, essentially consisting of or consisting of the sequence of SEQ ID NO: 1. "Essentially consisting of" relates to a nucleic acid encoding a protein consisting of the sequence encoded by SEQ ID NO: 1 and short C- and/or N-terminal sequences of at most 30, 25, 20, 15, 10, 9, 8, 7, 6, 5, 4, 3, 2, or 1 amino acids homologous or heterologous to the protein. These sequences may result from the genetic manipulations, e.g. the use of particular restriction sites, or may be needed for the purification of the protein, e.g. tags such as His-tag, Strep-tag, Arg-tag, c-myc-tag or Flag-Tag.

The feature "heterologous amino acid" or "amino acid heterologous to the protein" refers to any amino acid which is different from that amino acid located adjacent to a naturally occurring AS160 protein or a splice variant thereof, Therefore, the AS160- protein encompassing at least one heterologous amino acid refers to a protein which is different from any naturally occurring AS160 protein or splice variant thereof.

A functional characteristic of novel AS 160- protein can be any characteristic of the AS 160- protein as specified herein. Examples of such functional characteristics encompass, but are not limited to, e.g.: its tissue distribution, its implication in insulin-stimulated signal transduction modulation such as modulation and especially stimulation of insulin-stimulated glucose uptake, a modulation and especially stimulation of the phosphorylation of AKT, a modulation and especially stimulation of activity of PI3K (PI3-kinase) and MEKK/ERK kinases, modulation and especially stimulation of the translocation of GLUT4 to the plasma membrane, the interaction of AS160- protein with other proteins as known in the art, e.g. the interaction of AS160- protein with GLUT4 and any other of its functional characteristics, especially as depicted in the context of this application and the experimental results given below.

According to a preferred embodiment, the test system is in a cell. A cell-based system is advantageous, because it allows for easy amplification of the test system by propagating the cells and cellular mechanisms, e.g. signal transduction components downstream of insulin or downstream or upstream of AS160- protein, as these may be used in order to detect a signal indicative for altered glucose uptake of a cell.

Examples of cells suitable in the context of the present invention include without limitation L6 cells, HEK 293, 745-A, A-431, atrial myocytes, BxPC3, C5N, Caco-2, Capan-1, CC531, CFPAC, CHO, CHO K1, COS-1, COS-7, CV-1, EAHY, EAHY 926, F98, GH3, GP&envAM12, H-295 R, H-4-II-E, HACAT, HACAT A131, HEK, HEL, HeLa, Hep G2, High Five, Hs 766T, HT29, HUV-EC R24, HUV-EC-C, IEC 17, IEC 18, Jurkat, K 562, KARPAS-299, L 929, LIN 175, MAt-LYLU, MCF-7, MNEL, MRC-5, MT4, N64, NCTC 2544, NDCK II, Neuro 2A, NIH 3T3, NT2/D1, P19, primary neuronal cells, primary dendritic cells, primary human or mammalian myoblasts, primary keratinocytes, SF9, SK-UT-1, ST, SW 480, SWU-2 OS, U-373, U-937, rhabdomyosarcoma (RD) and Y-1. Other suitable cells are known to the one of skill in the art. One preferred example is a myoblast cell, such as L6.

Cells that are cultured directly from an animal or a person are known as primary cells. With the exception of some cell lines derived from tumours, most primary cell cultures have limited lifespan. After a certain number of population doublings cells undergo the process of senescence and stop dividing, while generally retaining viability.

An established or immortalised cell line has acquired the ability to proliferate indefinitely either through random mutation or deliberate modification, such as artificial expression of the telomerase gene. There are numerous well established cell lines representative of particular cell types and it is within the knowledge of the skilled person to select a suitable cell line.

Accordingly, in a preferred embodiment of the invention the cell is a cell line. A cell line is a population of cells propagated in culture that are derived from, and therefore genetically identical to, a single common ancestor cell. Preferred cell lines are L6 cells (see Examples), HEK 293 cells (primary human embryonic kidney), 3T3 cells (murine embryonic fibroblasts), CHO cells (Chinese hamster ovary), COS-7 cells (African green monkey cell line), HeLa cells (human epithelioid cervical carcinoma), JURKAT cells (human T-cell leukaemia), BHK 21 cell (hamster normal kidney, fibroblast), and MCF-7 cells (human breast cancer).

Preferred cell lines of skeletal muscle, liver or adipose tissue include without being limited thereto:
Skeletal muscle: C2C12 (mouse), L6 cell, such as L6-GLUT4myc (Wang et al, 1998) or L6-GLUT4myc-tetR, preferably DSM ACC2852 (see below).
Adipose tissue: brown adipocyte cell line HIB-1B), line F44-2A, those disclosed in US patent NO: 6,071,747.
Liver: BNL CL.2 (mouse, BALB/c), BNL SV A.8 (mouse), RLC-18 (rat) WRL 68.

A preferred cell line encompasses a gene coding for AS160 under the control of a tetracycline-responsive promoter system, such as L6-GLUT4myc-tetR-AS160_FL. Such a cell line was deposited under the Budapest Treaty on the International Recognition of the Deposit of Microorganisms for the Purposes of Patent Procedure at the "Deutsche Sammlung von Mikroorganismen und Zellkulturen GmbH" (DSMZ), Inhoffenstraße 7 B, 38124 Braunschweig, GERMANY under the accession number DSM ACC2942 on August 27, 2008 (referred to as L6-GLUT4myc-tetR-AS160_FL).

In order to screen for AS160-protein-mediated effects, the results obtained with the afore-mentioned cell line may be compared to those obtained with the same cell line, but lacking a introduction of a gene coding for AS160- protein (L6-GLUT4myc-tetR) which was deposited under the Budapest Treaty on the International Recognition of the Deposit of Microorganisms for the Purposes of Patent Procedure at the "Deutsche Sammlung von Mikroorganismen und Zellkulturen GmbH" (DSMZ), Inhoffenstraße 7 B, 38124 Braunschweig, GERMANY under the accession number DSM ACC2852 (referred to as L6-GLUT4myc-tetR) on June 20, 2007.

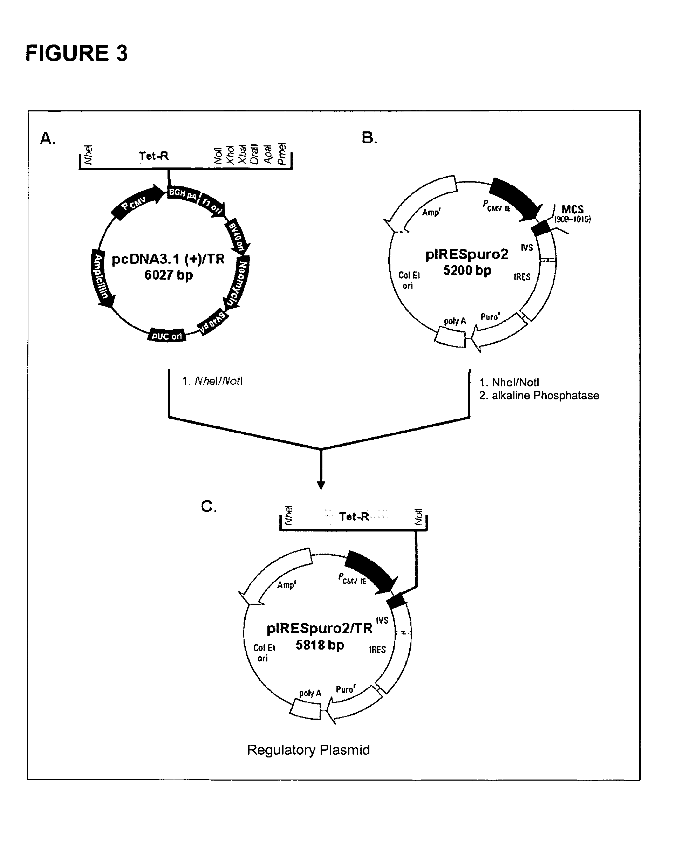

Both cell lines (L6-GLUT4myc-tetR-AS160 and L6-GLUT4myc-tetR) have been prepared as detailed in Example 2. Briefly summarized, the tetracycline-repressor (TR) was isolated from pCDNA3.1 (+)/TR (Invitrogen), cloned into the Nhel and Notl sites of pIPESpuro2 as shown in Figure 3 to obtain pIRESpuro2/TR which was transfected into L6 cells (rat skeletal muscle cells) stably expressing GLUT4myc (L6-GLUT4myc, described in Wang et al. 1998). For L6-GLUT4myc-tetR-AS160 cells, pCDNA5 vector (Invitrogen) containing the AS160 gene was additionally introduced into the cells.
For cultivation, cells may be grown and maintained at an appropriate temperature and gas mixture (typically, 37°C, 5% CO₂) in a cell incubator. Culture conditions vary widely for each cell type, and variation of conditions for a particular cell type can result in different phenotypes being expressed. Aside from temperature and gas mixture, the most commonly varied factor in culture systems is the growth medium. Recipes for growth media can vary in pH, glucose concentration, growth factors, and the presence of other nutrient components. Antibiotics can also be added to the growth media. Amongst the common manipulations carried out on culture cells are media changes and passaging cells. However, selection of suitable conditions is known to the skilled person.

The cell or cell line may be genetically engineered to include the test system of the invention. The test system may be located in a transient or stable transfected cell or cell line. The procedure for introducing a transgene into a recipient cell is called transfection. Transfection with DNA yields stable as well as unstable (transient) cells or cell lines. Transient cell lines reflect the survival of the transfected DNA in extrachromosomal form; stable cell lines result from the integration into the genome.

The transgenes can be introduced into the cells by a variety of means known to those knowledgeable in the art, and adapted to each cell type. Recombinant DNA cloning techniques well known in the art for introducing and expressing a nucleic acid molecule can be used to introduce and express the transgenes. Cells can be transfected using any appropriate means, including viral vectors, chemical transfectants, electroporation, calcium phosphate co-precipitation and direct diffusion of DNA. A suitable method for introducing a tests system into a recipient cell is detailed in Example 2 and may be adapted to the respective recipient cell.

As used herein, vectors are agents that transport the transgene into the cell and may include appropriate transcriptional and translational control signals such as a promoter. Vectors can be plasmid, viral or others known in the art, The promoter can be inducible or constitutive, general or cell specific, nuclear or cytoplasmic specific promoter. Selection of promoters, vectors and other elements is a matter of routine design within the level of ordinary skill in the art. Many such elements are described in the literature and are available through commercial suppliers. Usually, the method of transfer includes the transfer of a selectable marker to the cells. Suitable promoters and vectors are disclosed in the Examples and the present description.

In general, a cell line is transfected by any of the means mentioned above,
wherein the transgene is operatively linked to a selectable marker. Following transfection cells are grown e.g. for some days in enriched media and then switched to selective media. Transfected cells exhibit resistance to the selection and are able to grow, whereas non-transfected cells die in general. Examples for selective markers include puromycin, zeocin, neomycin (neo) and hygromycin B, which confer resistance to puromycin, zeocin, aminoglycoside G-418 and hygromycin, respectively, and/or any of the selective markers used in the Examples. However, other selection methods known to the skilled person may be also suitable.
In the step b) of the method of the present invention a test substance is identified as a substance altering glucose uptake of a cell by detecting a signal indicative for altered glucose uptake of a cell. The signal may be any suitable signal which is indicative for altered glucose uptake of a cell; however, the signal may by any component or part of the insulin-stimulated signal transduction relating to AS160-like protein. Particularly, it may be the degree of phosphorylation of AS160 or AKT, activity of PI3K (PI3-kinase) or MEKK/ERK kinases, translocation of GLUT4 to the plasma membrane or increase in glucose uptake of a cell.

Suitable methods for measuring the aforementioned components of the AS160-protein signal transduction pathway are known in the art and are also detailed in the Examples.

Preferably, the detectable signal is the amount of AS160- protein expressed in a cell, phosphorylated AKT, phosphorylated AS160- protein, GLUT4 translocation to the plasma membrane, GLUT4 distribution in a cell or glucose uptake by a cell. As could be shown in the examples, all these signals correlate with the glucose uptake of a cell, preferably a cell of an insulin-sensitive tissue.

The detectable signal may be the amount of AS160- protein in a cell, as the amount of this protein is indicative for glucose uptake. If the amount of this protein is increased, the glucose uptake of a cell, particularly an insulin-sensitive cell, is increased, too. Methods of determining the amount of a particular protein are known to the skilled person and include e.g. Western blotting and detection with specific antibodies, which may be carried out as detailed in the Examples. Specific antibodys are known, e.g. from Kane et al., 2002 and also provided in the Examples. Alternatively, an anti-AS160- monoclonal or polyclonal antibody may be produced in accordance with the knowledge of the skilled person and detected by enzyme-linked immunosorbent assay (ELISA), radioimmunoassay (RIA), and fluorescence activated cell sorting (FACS).

The preparation of suitable antibodies or functional fragments thereof is well known in the art, e.g. by immunizing a mammal, for example a rabbit, with AS 160-protein or a fragment thereof, where appropriate in the presence of, for example, Freund's adjuvant and/or aluminium hydroxide gels (see, for example, Diamond, B.A. et al. (1981) The New England Journal of Medicine: 1344-1349). The polyclonal antibodies which are formed in the animal as a result of an immunological reaction can subsequently be isolated from the blood using well known methods and, for example, purified by means of column chromatography. Suitable procedures to produce monoclonal antibodies are well known in the art as well (see e.g. Winter, G. & Milstein, C. (1991) Nature, 349, 293-299 and literature for standard methods listed below). In the context of present invention, the term antibody or antibody fragment comprises also recombinant antibodies or antigen-binding parts thereof, e.g. chimaeric, humanized, multifunctional, bispecific, oligospecific or single-stranded antibodies or antibody F(ab) or F(ab)₂ fragments (see, e.g. EP-B1-0 368 684, WO 88/01649, WO 93/06213, WO 98/24884, US 4,816,567 or US 4,816,397).

A variety of other techniques known in the art can be used to quantify the amount of a given protein. These include, but are not limited to immunological techniques such as an ELISA or RIA, or quantitative analytical techniques such as spectroscopy or flame chromatography. Alternatively, the amount of AS160-mRNAs could be determined by a hybridization method or a nucleic acid amplification method instead of the amount of protein. Such methods are known to the artisan and include the dot blot hybridization method, the Northern hybridization method or the RT-PCR method.

An alternative detectable signal may be the amount of phosphorylated AKT and/or phosphorylated AS160- protein, as the degree of phosphorylation of these proteins is indicative for glucose uptake. If the amount or degree of phosphorylation is increased, the glucose uptake of a cell, particularly an insulin-sensitive cell, is increased, too. Methods of determining the amount or degree of phosphorylation are known to the skilled person and include the use of antibodies specific for these phosphorylated proteins as detailed in the Examples.

A further detectable signal may be GLUT4 translocation to the plasma membrane or GLUT4 distribution in a cell, as the degree of translocation of GLUT4 is indicative for glucose uptake. If the amount or GLUT4 in the plasma membrane is increased, the glucose uptake of a cell, particularly an insulin-sensitive cell, is increased, too. Methods of determining GLUT4 translocation to the plasma membrane or GLUT4 distribution in a cell are known to the skilled person and include the use myc-tagged GLUT4 in an In-cell- Western technique or in a ACUMEN technique. These methods may be carried out as detailed in the Examples.

Alternatively, glucose uptake of a cell could be determined, e.g. by using labeled glucose or a labeled glucose derivative. Suitable labels include e.g. detectable tags, radio-active isotopes such as ³H or ¹⁴C or fluorescence markers. Such labeled glucose or a labeled glucose derivatives include without limitation 2-fluoro-2-deoxy-D-glucose, 2-deoxy[¹⁴C] glucose and [¹⁴C]methylglucose. Preferably, radio-labeled 2-deoxyglucose is used. This method may be carried out as detailed in the Examples.
As detailed above, the method of the invention may be used in order to test as substance under high glucose condition, which better reflects the situation in a patient suffering from diabetes. High glucose conditions are those with elevated glucose concentration. The normal / safe level for glucose in the blood of a human is between 3.5 and 7.8 mM. Accordingly, a high glucose condition is a condition with glucose concentration above the normal level. Particularly, the glucose concentration used for the method of the invention may be at least 10 mM, preferably at least 15 mM, more preferably at least 25 mM glucose.

The substance tested with the method of the invention may be any test substance or test compound of any chemical nature. It may be already known as a drug or medicament for a disease other than type 2 diabetes. Alternatively, it may be a known chemical compound not yet known to have a therapeutic effect. In another embodiment the chemical compound may be a novel or so far unknown chemical compound.

In another embodiment of the screening methods of the invention, the test substance is provided in the form of a chemical compound library. Chemical compound libraries include are plurality of chemical compounds and have been assembled from any of multiple sources, including chemically synthesized molecules and natural products, or have been generated by combinatorial chemistry techniques. They are especially suitable for high throughput screening. They may be comprised of chemical compounds of a particular structure or compounds of a particular creature such as a plant. In the context with the present invention the chemical compound library is preferably a library comprising proteins and polypeptides or small molecules.

Advantageously, the method of the present invention is carried out in a robotics system e.g, including robotic plating and a robotic liquid transfer system, e.g. using microfluidics, i.e. channelled structured.

In another embodiment of the present invention, the method is carried out in form of a high-through put screening system. In such a system advantageously the screening method is automated and miniaturized; in particular it uses miniaturized wells and microfluidics controlled by a roboter. High-throughput screening (HTS), is a method for scientific experimentation especially used in drug discovery and relevant to the fields of biology and chemistry.

HTS allows a researcher to effectively conduct millions of biochemical, genetic or pharmacological tests in a short period of time, often through a combination of modem robotics, data processing and control software, liquid handling devices, and sensitive detectors. Through this process one can rapidly identify active compounds which modulate a particular biomolecular pathway; particularly a substance altering the glucose uptake of a cell.

In essence, HTS uses an approach to collect a large amount of experimental data on the effect of a multitude of substances on a particular target in a relatively short time. A screen, in this context, is the larger experiment, with a single goal (usually testing a scientific hypothesis), to which all this data may subsequently be applied.

For HTS, cells comprising AS160- protein or a nucleic acid coding for the same may be seed in a tissue plate, such as a multi well plate, e.g. a 96-well plate. Then the cell in the plate is contacted with the test substance for a time sufficient to stimulate and generate a suitable detectable signal as defined above. The test substance may be different from well to well across the plate. After incubation time has passed to allow generation of the signal, measurements are taken across all the plate's wells, either manually or by a machine.

Manual measurements may be necessary when the researcher is using microscopy to (for example) seek changes the wells' test compounds, looking for effects that a computer could not easily determine by itself. Otherwise, a specialized automated analysis machine can run a number of experiments on the wells (such as analyzing light of a particular frequency). In this case, the machine outputs the result of each experiment e.g. as a grid of numeric values, with each number mapping to the value obtained from a single well.

Depending upon the results of this first assay, the researcher can perform follow up assays within the same screen by using substances similar to those identified as active (i.e. altering glucose uptake of a cell) into new assay plates, and then rerunning the experiment to collect further data, optimize the structure of the chemical compound to improve the effect of the compound on the cell.

Automation is an important element in HTS's usefulness. A specialized robot is often responsible for much of the process over the lifetime of a single assay plate, from creation through final analysis. An HTS robot can usually prepare and analyze many plates simultaneously, further speeding the data-collection process.

A further subject of the invention relates to a test system for the identification of a substance for improving glucose uptake into a cell, the test system comprising
a gene coding for the AKT substrate 160 kDa- protein (AS160- protein) or functional variant thereof; and
an inducible promoter providing controllable expression of the gene,
wherein the activation of AS160- protein effects a detectable signal.

It is noted that all features of this test system may be further defined as detailed in connection with the method of the invention.

The test system is particularly useful as it allows for identification of a substance improving (i.e. increasing) glucose uptake of a cell or as a model for studying type 2 diabetes. The combination of AS160- protein and an inducible promoter providing controllable expression of the gene allows for determining effects in identical cells with and without AS160- protein. Accordingly, differences in signaling obtained in cells expressing AS160- protein in comparison to those not expressing AS160- protein may be assigned to AS160- protein. As AS160- protein may be used to identify new potential drugs for type 2 diabetes (as detailed above) or as a key protein in the main insulin-sensitive tissues, these test system may be used to obtain news insights in the pathophysiology and therapy of type 2 diabetes.

Preferably, the test system of the invention is located in a cell, particularly a genetically engineered cell. The cell any be any of the cells disclosed in the context of the method of the invention. Particularly the gene and/or the promoter may be introduced into the genetically engineered cell.

The test system of the invention comprises an inducible promoter providing controllable expression of the gene. Controllable expression of the gene means that the expression can be induced or repressed upon a chemical or physical stimulus to the test system which can be applied as intended by the investigator or experimenter.

Promoters represent critical elements that can work in concert with other regulatory regions (enhancers, silencers, boundary elements/insulators) to direct the level of transcription of a given gene. An inducible promoter is activated in response to either the presence of a particular compound, i.e. the inducer (chemical stimulus) or to a defined physical condition, e.g. elevated temperature (physical stimulus). Inducible promoters are a very powerful tool in genetic engineering because the expression of genes operably linked to them can be turned on or off as desired.

There are a series of chemically-regulated promoters, including promoters whose transcriptional activity is regulated by the presence or absence of alcohol, tetracycline, steroids, metal and other compounds. Physically-regulated promoters include promoters whose transcriptional activity is regulated by the presence or absence of light and low or high temperatures.

Preferably, chemically-regulated promoters should be derived from organisms distant in evolution to the cell where its action is required. Thus, promoters to be used in mammalian cells are mostly derived from organisms such as yeast, E. coli or Drosophila. Particular examples are alcohol-regulated promoter system (alcohol dehydrogenase I (alcA) gene promoter and the transactivator protein AlcR); tetracycline-regulated promoter system (tetracycline repressor protein (TetR), tetracycline operator sequence (tetO), tetracycline transactivator fusion protein (tTA), which is the fusion of TetR and a herpes simplex virus protein 16 (VP16) activation sequence, the promoter system disclosed in Example 2); steroid-regulated promoter systems (steroid-responsive promoter, e.g. promoters based on the rat glucocorticoid receptor (GR) or promoters based on the human estrogen receptor (ER)); or metal-regulated promoters derived from metallothionein genes from yeast, mouse and human.

However, the inducible promoter is preferably a tetracycline-inducible promoter, more preferably the promoter system as described in Example 2.

A further aspect of the invention relates to the use of a tests system comprising AS160- protein for the identification of a substance altering, particularly improving, glucose uptake into a cell as already detailed above in the context of the method are test system of the invention. The test system used may be further specified as described in above with respect to the method or test system of the invention.

Also in accordance with the above disclosure AS160- protein may be used in a model for type 2 diabetes, wherein the above details with respect to the method or test system of the invention are to be applied accordingly.

According to another embodiment, the invention concerns the use of a cell heterologously expressing a polypeptide consisting or essentially consisting of the amino acid sequence according to SEQ ID NO: 2 or being encoded by a sequence according to SEQ 1D NO: 1 or a cell stably or transiently transfected with a polynucleotide consisting of or essentially consisting of polynucleotide sequence according to SEQ ID NO: 1 or encoding a polypeptide according to SEQ ID NO: 2.

The cell can be any procaryotic or eucaryotic cell capable of being stably or transiently transfected with a nucleic acid vector and of expressing a heterologous gene. These comprise principally primary cells as well as cells from a cell culture, preferably a eucaryotic cell culture comprising cells derived either from multicellular organisms and tissue (such as HeLa, CHO, COS, SF9 or 3T3 cells) or from single cell organisms such as yeast (e.g. s. pombe or s. cerevisiae), or a procaryotic cell culture, preferably Pichia or E. coli. Cells and samples derived from tissue can be gained by well-known techniques, such as taking of blood, tissue punction or surgical techniques.

Another aspect of the invention concerns the use of a siRNA (small inhibitory RNA) capable of negatively interfering with expression and/or activity of AS160, e.g. specific for or in part complementary to at least a part of the DNA sequence SEQ ID NO:1, for the lowering or inhibition (partial or complete) of the Glucose-uptake of a cell.

The term "siRNA" refers to small inhibitory RNAs that induce the RNA interference (RNAi) pathway (for the RNAi interference pathway, see e.g. Elbashir et al., Genes and Development (2001) 15: 188-200, Tuschl. et al., (1999), Genes and Development, 13: p. 3191-3197 or Zamore et al, Cell (2000) vol.101, p.25-33. In the context of present invention the term "siRNA" comprises duplexes of two separate strands, as well as single strands that can form hairpin strucures comprising a duplex region duplexes, so-called shRNAs - short hairpin RNAs. siRNA molecules can vary in length (in general 15 to 35, 18 to 30 or 20 to 25 nucleotides in length); however, the choice of the appropriate length is well known in the art. Moreover, siRNAs can vary in their degree of complementarity to thir target mRNA in the antisense strand. The choice of the appropriate degree of complementarity is also well known in the art. siRNA may have unpaired overhanging bases on the 5' and/or the 3' end of the sense strand and/or the antisense strand.
Design and preparation of siRNAs for a given cDNA sequence are well known in the art see, for example Elbashir et al. (2001) Nature 411: 494-498 (see especially p.497, right column, for preparation of siRNA and siRNA transfection into cells), and Tuschl et al. (1999) Genes and Development 13: 3191-3197).
Methods for application of siRNA or shRNA include chemically synthesized or *in vitro* transcribed siRNA, e.g. duplex or shRNA, which are than to be transfected or injected into cells or transgenic animals. SiRNA/shRNA can also be expressed from expression vectors or PCR products in cells or transgenic animals, wherein the term expression vector refers to any kind of vector system useful for driving expression of siRNAs or shRNAs and comprises shuttle vectors as well as viral, such as retro-and lentiviral vectors, as well known in the art.

According to another aspect, the invention refers to the use of the AS 160- protein or the nucleic acid sequence thereof, for generating a siRNA and/or a shRNA able to negatively interfere with Glucose uptake of a cell.
The following figures and examples shall illustrate the present invention, but should not be understood as limiting the scope of the invention.

### List of References cited:

Bruss,M.D., Arias,E.B., Lienhard,G.E., and Cartee,G.D. (2005). Increased phosphorylation of Akt substrate of 160 kDa (AS160) in rat skeletal muscle in response to insulin or contractile activity. Diabetes. 54, 41-50.
Deshmukh,A., Coffey,V.G., Zhong,Z., Chibalin,A.V., Hawley,J.A., and Zierath,J.R. (2006). Exercise-Induced Phosphorylation of the Novel Akt Substrates AS160 and Filamin A in Human Skeletal Muscle. Diabetes. 55, 1776-1782.
Ding,V.D., Qureshi,S.A., Szalkowski,D., Li,Z., Biazzo-Ashnault,D.E., Xie,D., Liu,K., Jones,A.B., Moller,D.E., and Zhang,B.B. (2002). Regulation of insulin signal transduction pathway by a small-molecule insulin receptor activator. Biochem J. 367, 301-306.
Eguez,L., Lee,A., Chavez,J.A., Miinea,C.P., Kane,S., Lienhard,G.E., and McGraw,T.E. (2005). Full intracellular retention of GLUT4 requires AS160 Rab GTPase activating protein. Cell Metab. 2, 263-272.
M. Larance, G.Ramm, J.Stockli, E.M.van Dam, S.Winata, V.Wasinger, F.Simpson, M.Graham, J.R.Junutula, M.Guilhaus, D.E.James, J Biol.Chem. 280 (2005) 37803.
S. Ishikura, P.J.Bilan, A.Klip, Biochem.Biophys.Res.Commun. 353 (2007) 1074.
W.G. Roach, J.A.Chavez, C.P.Miinea, G.E.Lienhard, Biochem.J. 403 (2007) 353.
Treebak,J.T., Glund,S., Deshmukh,A., Klein,D.K., Long,Y.C., Jensen,T.E., Jorgensen,S.B., Viollet,B., Andersson,L., Neumann,D., Wallimann,T., Richter,E.A., Chibalin,A.V., Zierath,J.R., and Wojtaszewski,J.F. (2006), AMPK-mediated AS160 phosphorylation in skeletal muscle is dependent on AMPK catalytic and regulatory subunits. Diabetes. 55, 2051-2058.
Tsiani,E., Bogdanovic,E., Sorisky,A., Nagy,L., and Fantus,I.G. (1998). Tyrosine phosphatase inhibitors, vanadate and pervanadate, stimulate glucose transport and GLUT translocation in muscle cells by a mechanism independent of phosphatidylinositol 3-kinase and protein kinase C. Diabetes. 47, 1676-1686.
R.T. WatsonJ.E.Pessin, Trends Biochem Sci. 31 (2006) 215.Yuan,J.S., Reed.A., Chen,F., and Stewart,C.N., Jr. (2006). Statistical analysis of real-time PCR data. BMC Bioinformatics. 7:85., 85.
Bouzakri,K. and Zierath,J.R. (2007). MAP4K4 gene silencing in human skeletal muscle prevents TNF-alpha -induced insulin resistance. J Biol Chem. 282, 7783-7789
Bowen,W.P. and Wylie,P.G. (2006). Application of laser-scanning fluorescence microplate cytometry in high content screening. Assay. Drug Dev. Technol. 4, 209-221.
Ciaraldi,T.P., Carter,L., Rehman,N., Mohideen,P., Mudaliar,S., and Henry,R.R. (2002). Insulin and insulin-like growth factor-1 action on human skeletal muscle: preferential effects of insulin-like growth factor-1 in type 2 diabetic subjects. Metabolism. 51, 1171-1179.
DeSilva,D.R., Jones,E.A., Favata,M.F., Jaffee,B.D., Magolda,R.L., Trzaskos,J.M., and Scherle,P.A. (1998). Inhibition of mitogen-activated protein kinase kinase blocks T cell proliferation but does not induce or prevent anergy. J Immunol. 160, 4175-4181.
Elbashir S.M., Harborth J., Lendeckel W., Yalcin A., Weber K., and Tuschl T. (2001). Duplexes of 21-nucleotides RNAs mediate RNA interference in mammalian cell culture. Nature. 411. 494-498
Greene,E.L., Nelson,B.A., Robinson,K.A., and Buse,M.G. (2001). alpha-Lipoic acid prevents the development of glucose-induced insulin resistance in 3T3-L1 adipocytes and accelerates the decline in immunoreactive insulin during cell incubation. Metabolism. 50, 1063-1069.
Hu,Y., Qiao,L., Wang,S., Rong,S.B., Meuillet,E.J., Berggren,M., Gallegos,A., Powis,G., and Kozikowski,A.P. (2000). 3-(Hydroxymethyl)-bearing phosphatidylinositol ether lipid analogues and carbonate surrogates block PI3-K, Akt, and cancer cell growth. J Med Chem. 43, 3045-3051.
Kane,S., Sano,H., Liu,S.C., Asara,J.M., Lane,W.S., Garner,C.C., and Lienhard,G.E. (2002). A method to identify serine kinase substrates. Akt phosphorylates a novel adipocyte protein with a Rab GTPase-activating protein (GAP) domain. J Biol. Chem. 277, 22115-22118.
Kansson.H.K., Hallsten,K., Bjomholm,M., Tsuchida,H., Chibalin,A.V., Virtanen,K.A., Heinonen,Q.J., Lonnqvist,F., Nuutila,P., and Zierath,J.R. (2005a). Effects of metformin and rosiglitazone treatment on insulin signaling and glucose uptake in patients with newly diagnosed type 2 diabetes: a randomized controlled study. Diabetes. 54, 1459-1467.
Karlsson,H.K., Zierath,J.R., Kane,S., Krook,A., Lienhard,G.E., and Wallberg-Henriksson,H. (2005b). Insulin-stimulated phosphorylation of the Akt substrate AS160 is impaired in skeletal muscle of type 2 diabetic subjects. Diabetes. 54, 1692-1597.
Nelson,B.A., Robinson,K.A., and Buse,M.G. (2002). Defective Akt activation is associated with glucose- but not glucosamine-induced insulin resistance. Am. J Physiol Endocrinol Metab. 282, E497-E506.
Okada,T., Kawano,Y., Sakakibara,T., Hazeki,O., and Ui,M. (1994). Essential role of phosphatidylinositol 3-kinase in insulin-induced glucose transport and antilipolysis in rat adipocytes. Studies with a selective inhibitor wortmannin. J Biol. Chem. 269,3568-3573.
Sano,H., Kane,S., Sano,E., Miinea,C,P., Asara,J.M., Lane,W.S., Gamer,C.W., and Lienhard,G.E. (2003). Insulin-stimulated phosphorylation of a Rab GTPase-activating protein regulates GLUT4 translocation. J Biol. Chem. 278, 14599-14602.
Tuschl T., Zamore PD., Lehmann R., Bartel DP. and Sharp PA (1999). Targeted mRNA degradation by double-stranded RNA in vitro. Genes and Development 13. 3191-3197
Voss,M.D., Beha,A., Tennagels,N., Tschank,G., Herling,A.W., Quint,M., Gerl,M., Metz-Weidmann,C., Haun,G., and Korn,M. (2005). Gene expression profiling in skeletal muscle of Zucker diabetic fatty rats: implications for a role of stearoyl-CoA desaturase 1 in insulin resistance. Diabetologia. 48, 2622-2630.
Walgren,J.L., Vincent,T.S., Schey,K.L., and Buse,M.G. (2003). High glucose and insulin promote O-GlcNAc modification of proteins, including alpha-tubulin. Am. J Physiol Endocrinol Metab. 284, E424-E434.
Wang Q., Khayat Z., Kishi Y., Ebina Y., and Klip A. (1998). GLUT4 translocation by insulin in intact muscle cells: detection by a fast and quantitative assay. FEBS Lett. 427 (2), 193-197
Yuan,J.S., Reed,A., Chen,F., and Stewart,C.N., Jr. (2006). Statistical analysis of real-time PCR data. BMC Bioinformatics. 7:85., 85.
Zeigerer,A., McBrayer,M.K., and McGraw,T.E. (2004). Insulin stimulation of GLUT4 exocytosis, but not its inhibition of endocytosis, is dependent on RabGAP AS160. Mol. Biol. Cell. 15, 4406-4415.

If not indicated otherwise, standard laboratory methods were or can be performed according to standard procedures known in the art, e.g. as outlined the following laboratory standard literature: Sambrook et al. (1989) Molecular Cloning: A Laboratory Manual. Second edition. Cold Spring Harbor Laboratory Press, Cold Spring Harbor, NY. 545 pp;
Current Protocols in Molecular Biology; regularly updated, e.g. Volume 2000; Wiley & Sons, Inc; Editors: Fred M. Ausubel, Roger Brent, Robert Eg. Kingston, David D. Moore, J.G. Seidman, John A. Smith, Kevin Struhl. Current Protocols in Human Genetics; regularly updated; Wiley & Sons, Inc; Editors: Nicholas C. Dracopoli, Honathan L. Haines, Bruce R. Korf, Cynthia C. Morton, Christine E. Seidman, J.G. Seigman, Douglas R. Smith. Current Protocols in Protein Science; regularly updated; Wiley & Sons, Inc; Editors: John E. Coligan, Ben M. Dunn, Hidde L. Ploegh, David W. Speicher, Paul T. Wingfield. Molecular Biology of the Cell; third edition; Alberts, B., Bray, D., Lewis, J., Raff, M., Roberts, K., Watson, J.D.; Garland Publishing, Inc. New York & London, 1994;
Short Protocols in Molecular Biology, 5th edition, by Frederick M. Ansubel (Editor), Roger Brent (Editor), Robert E. Kingston (Editor), David D. Moore (Editor), J.G. Seidman (Editor), John A. Smith (Editor), Kevin Struhl (Editor), October 2002, John Wiley & Sons, Inc., New York"
Gene targeting: A Practice Approach, 2nd Ed., Joyner AL, ed. 2000. IRL Press at Oxford University Press, New York;

### Legend to the FIGURES

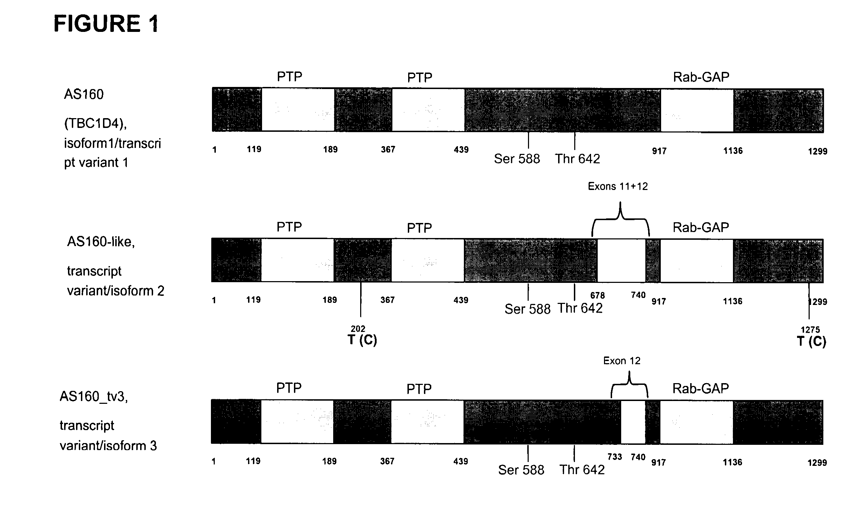

Figure 1 shows a comparison of the three different isoforms of AS160. Isoform 2 as well as isoform 3 have been identified on the basis of quantitative RT PCR (Taqman) using primers SEQ ID NO 7, 8, 9 for isoform 2. AS160, isoform 1 (amplified with primers SEQ ID NO 19, 20 and 21) is depicted in the upper lane, the isoform 2, which lacks the exons 11 and 12 in the middle lane. Isoform 3, which lacks exon 12, is shown in the lowest lane (amplified with primers SEQ ID NO 16, 17, 18). Phosphorylation sites Ser-588 and Thr-642 (underlined) and mismatches T202 and T1275 (italic) are shown. Phosphorylation sites are shown underneath.
Figure 2 (A and B) shows expression of AS160-protein and the two new isoforms (Taqman). Using specific primers (Primers SEQ ID NO 4, 5,6 and/or 19, 20, 21) for AS160-protein (here called isoform 1), primers SEQ ID NO 7,8, 9 for isoform 2, and primers 16, 17, 18 for isoform 3), the expression of the three different isoforms was examined. mRNA levels of AS160 isoforms are normalized against expression of endogenous RPL37a mRNA (amplified with primers SEQ ID NO 10, 11, 12). Data are representative for five different human donors.
Figure 3 shows a schematic presentation of cloning strategy for cloning of AS160. The tet-repressor derived from pCDNA3.1 (+)/TR was cloned into Nhel and Notl sites of pIRESpuro2 generating pIRF-Spuro2/TR.
Figure 4: Functionality of the T-Rex system. L6-GLUT4myc cells were transiently transfected with 2 µg pCDNA5/TO/GFP. In the absence of doxycycline only a minor number of cells express GFP (A). Addition of doxycycline (1µg/ml) increases the number of GFP positive cells up to about 10 fold (B).
Figure 5: Expression of AS160 in L6-GLUT4myc cells is tet-inducible. RIPA extracts were analyzed with SDS-PAGE and western blot analysis. Cells were incubated in the absence of doxycycline (- dox) or in the presence of doxycycline (+ dox) for 48 hours. Expression of AS160 was detected with a specific AS160 antibody (Upstate, 07-741). To confirm equal loading the blot was probed with an **actin antibody.**
Figure 6: Effect of AS160 protein on insulin stimulated glucose uptake. Uptake of 2-deoxyglucose is measured in response to insulin. Insulin was incubated for 20 minutes. Standard deviations are representative for 8 reading points.

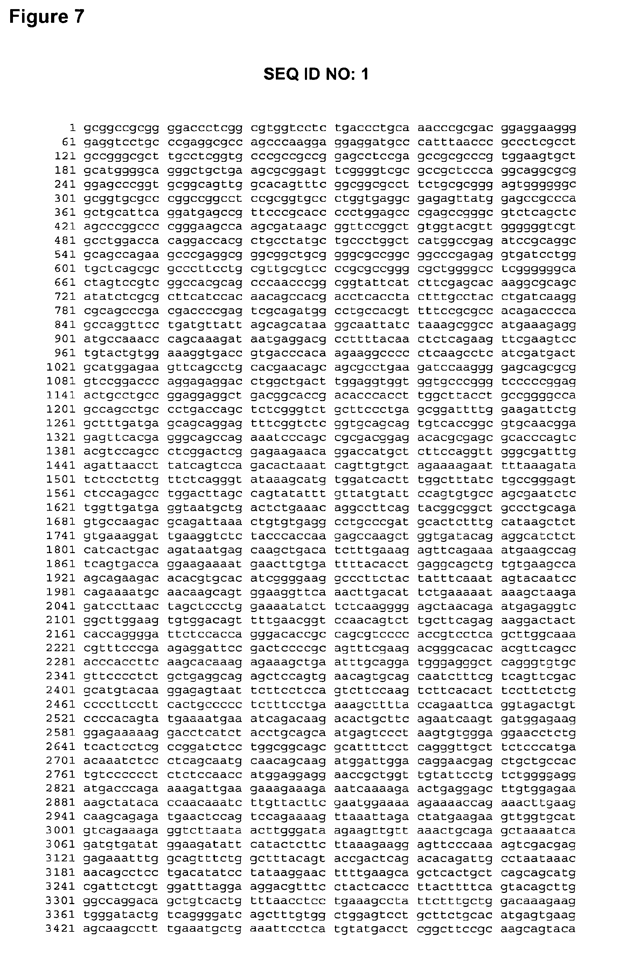

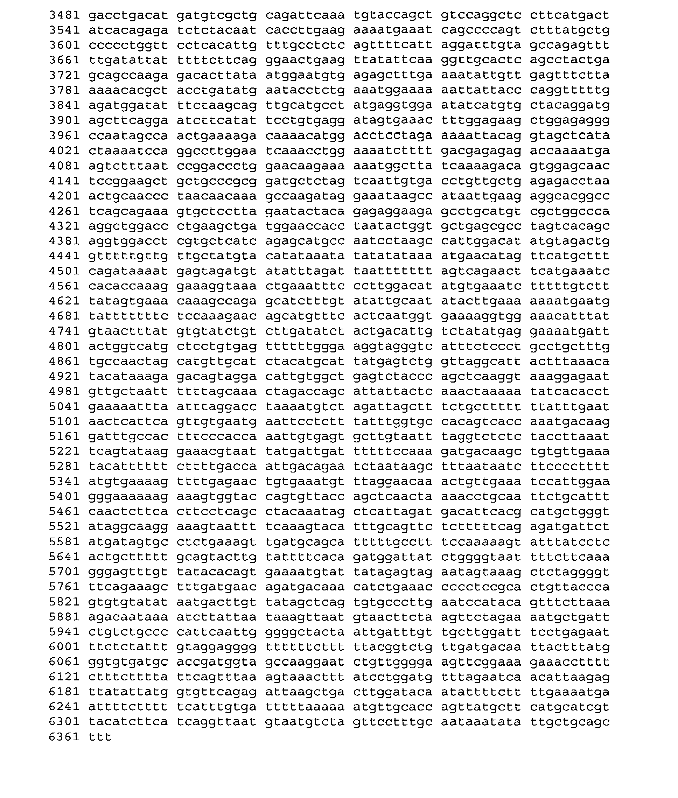

Figure 7 shows the coding sequence of human AS160 (NM_014832, SEQ ID NO:1).

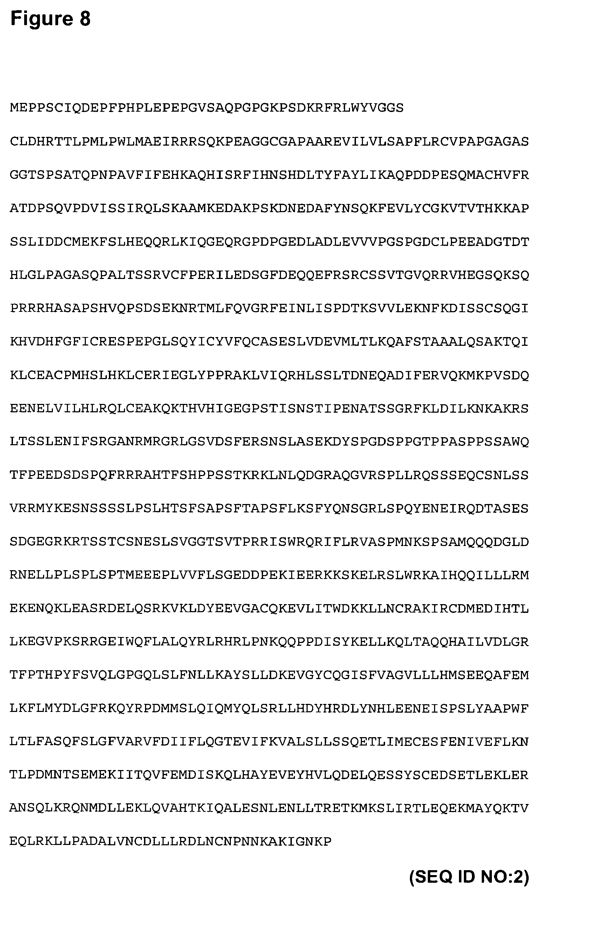

Figure 8 shows the translated protein sequence of human AS 160 (NP_055647, SEQ ID NO:2).

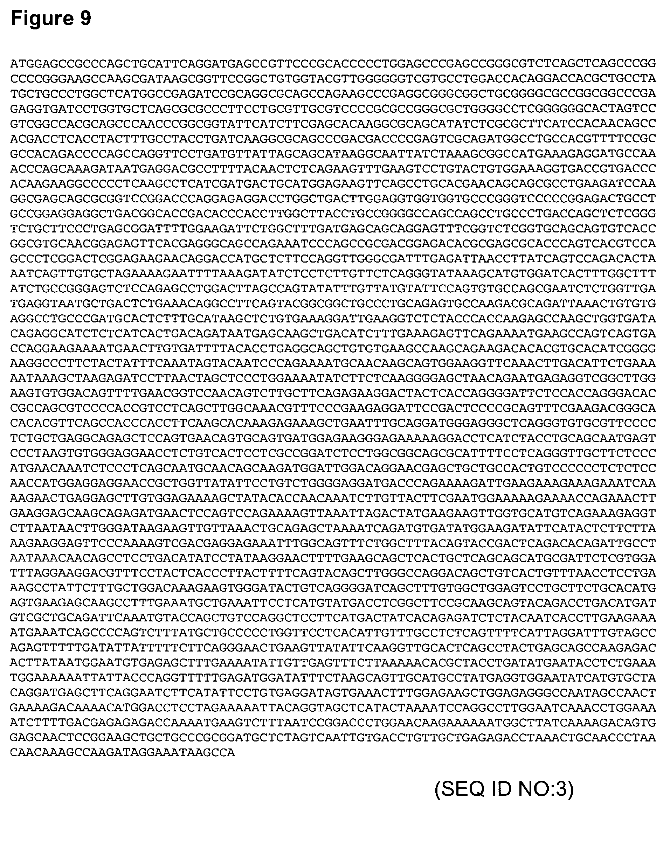

Figure 9 shows the insert for cloning of AS160 (isoform 1)(SEQ ID NO.3).

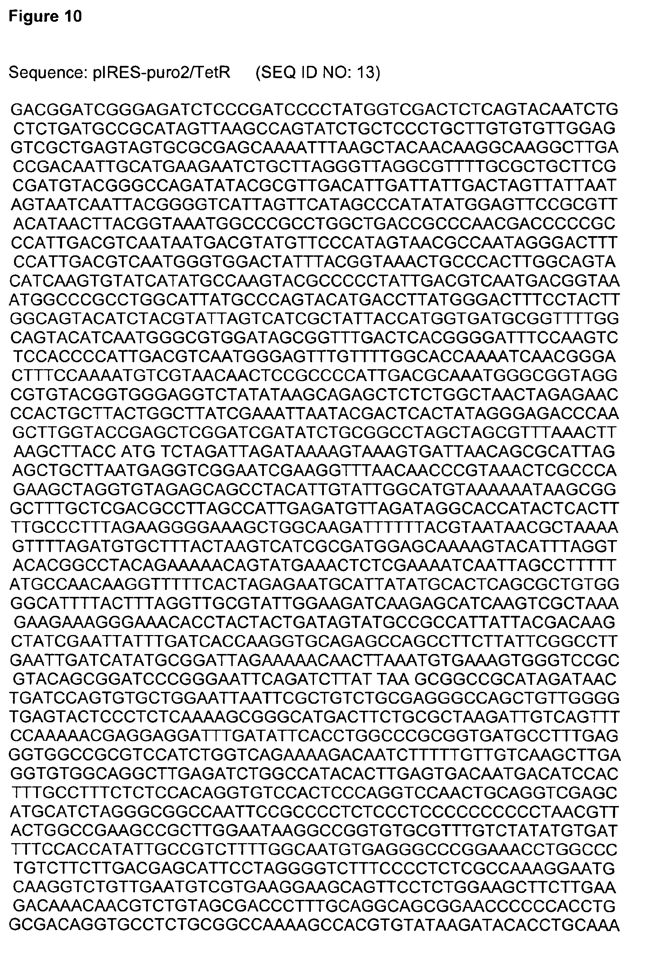

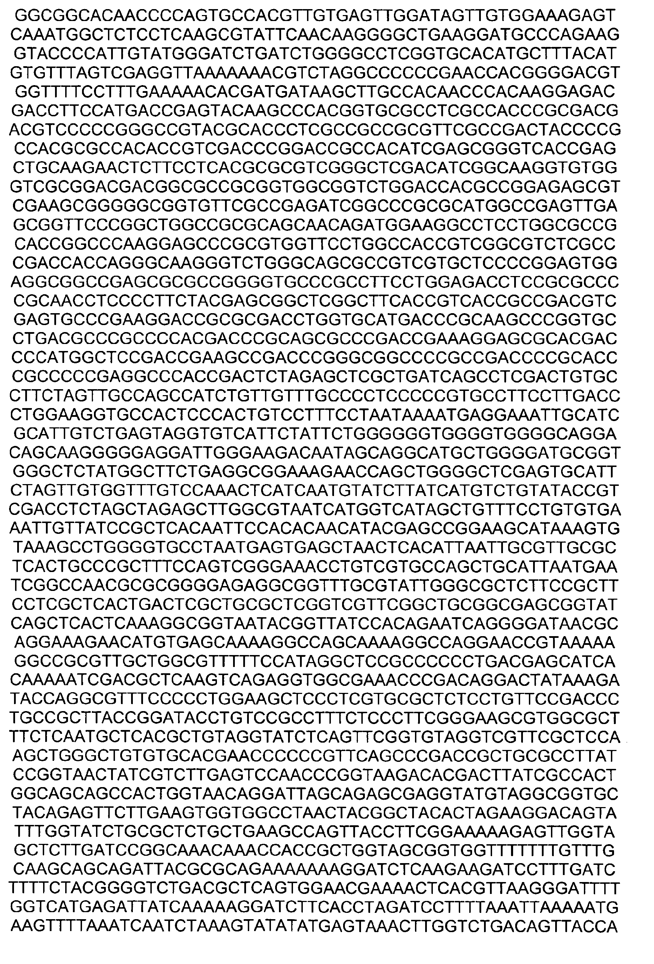

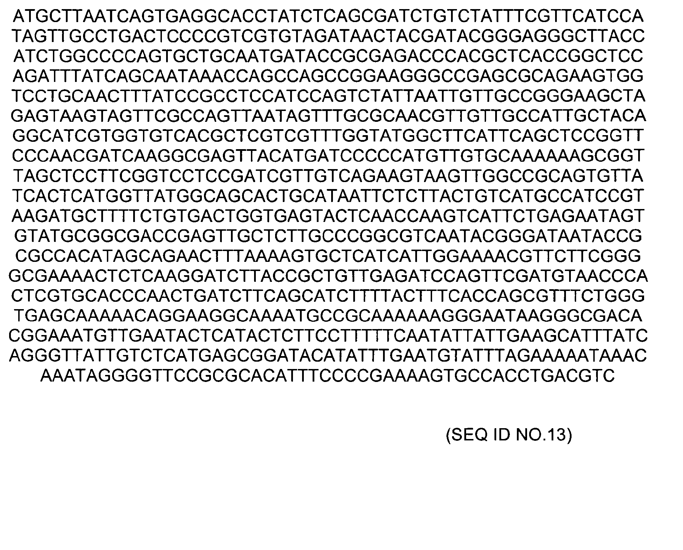

Figure 10 shows the sequence of pIRES-puro2/TetR (SEQ ID NO.13).

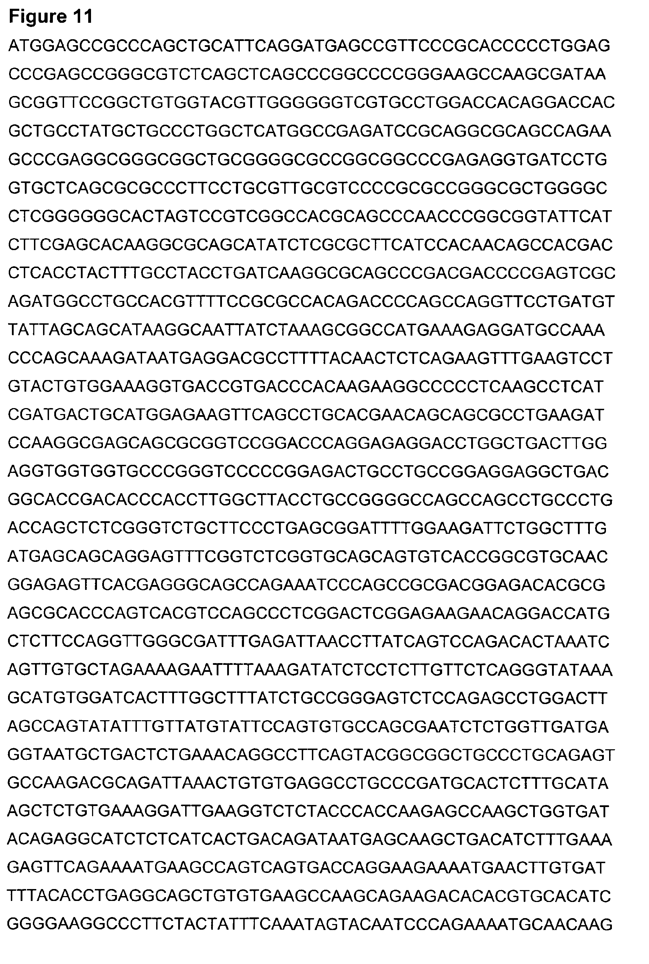

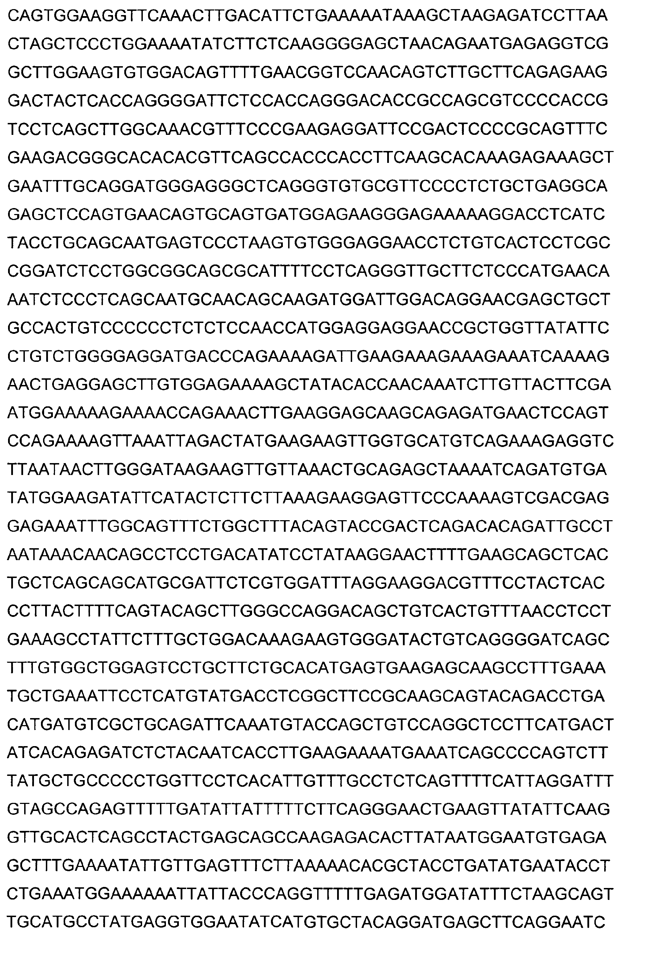

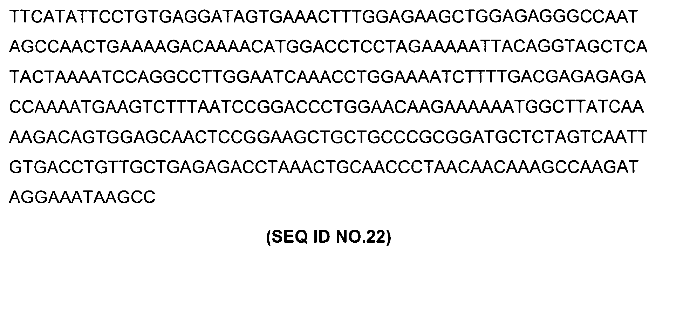

Figure 11 shows the coding sequence of novel isoform 2 (SEQ ID NO.22).

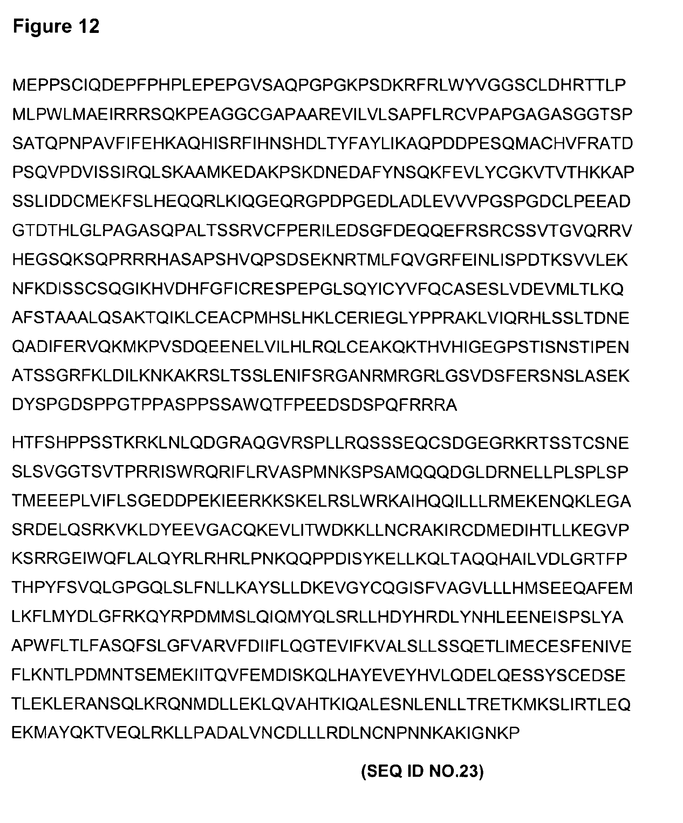

Figure 12 shows the translated protein sequence of novel isoform 2 (SEQ ID NO.23).

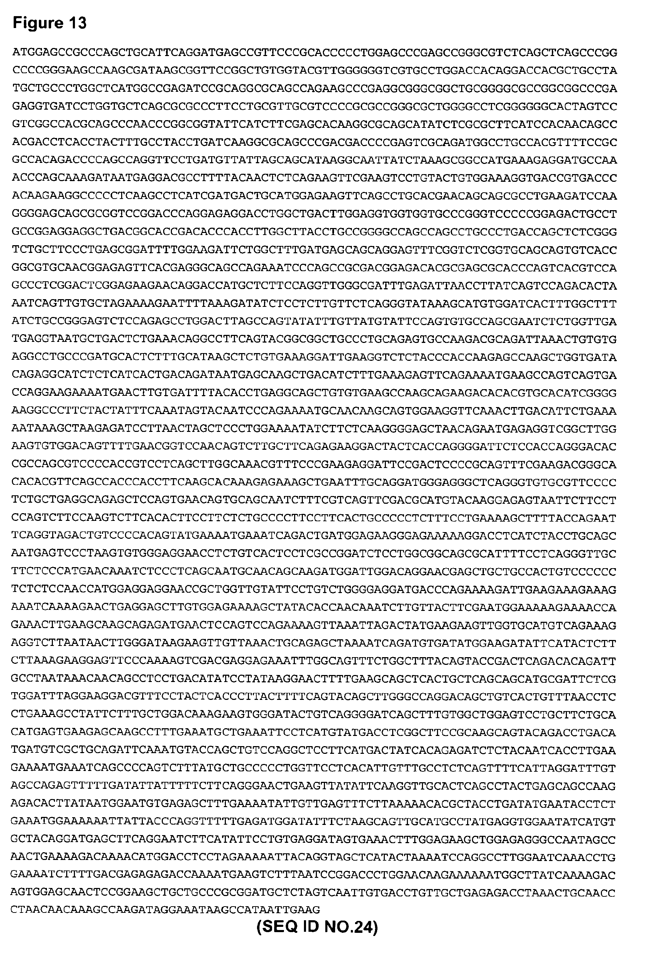

Figure 13 shows the coding sequence of novel isoform 3 (SEQ ID NO.24).

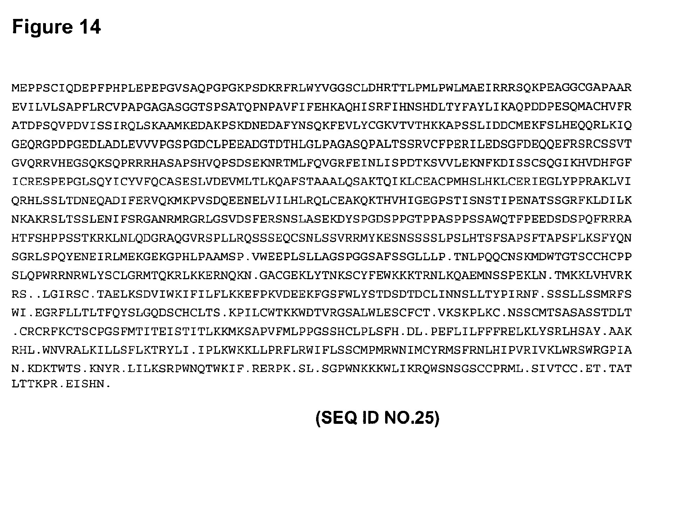

Figure 14 shows the translated protein sequence of novel isoform 3 (SEQ ID NO.25).

### EXAMPLES

### Example 1: Expression of AS160-protein and novel isoforms 2 and 3 in different tissues

Figure 1 presents a schematic overview of AS160 and two novel isoform. The expression of these different AS160-isoforms was investigated with quantitative RT-PCR in different tissues. Commercially available RNAs of five different human donors were reverse transcribed and examined using Taqman PCR with specific primer pairs (for AS160 SEQ ID NO: 4, 5 and 6, (or alternatively 19,20 and 21).

For this, aliquots of total cellular RNA were subjected to first-strand DNA synthesis. Reverse-transcribed cDNA was used as a template for amplification. A common probe was used to determine the overall AS160 expression in insulin-sensitive tissue (adipose, muscle, liver, heart, brain). Endogenous mRNA expression of the ribosomal gene RPL37a (Homo sapiens ribosomal protein L37a, mRNA, cDNA clone MGC:26772) was used to normalize mRNA levels (SEQ ID NO: 10, 11 and 12). Based on specific primer pairs the expression of the distinct isoforms could be distinguished. Relative mRNA expression methods were calculated with the deltadelta CT method (Yuan et al., 2006).

The following primers and probes were used:

| **Specificity** | **Sequence** | **Primer No/ SEQ ID NO:** |
|---|---|---|
| **AS 160 (isoform 1)** | For 5'-ATTCAGGTAGACTGTCCCCACAGTAT | 19 |
| | Rev 5'-CCTTCTCCATCACTTGATTCTGAAG | 20 |
| | Probe: FAM-MGBNFQ 5'-ATGAAATCAGACAAGACACTG | 21 |
| | For 5'- CATACTCTTCTTAAAGAAGGAGTTCCCA | 4 |
| | Rev 5'- CTGTGTCTGAGTCGGTACTGTAAAGC | 5 |
| | Probe: FAM-TAMRA 5'- CAGAAACTGCCAAATTTCTCCTCGTCGACT | 6 |
| **PCR cloning of AS160 (isoform1)** | For 5'-GGAGGAGGATGCCCATTTAAC | 14 |
| | Rev 5'-TGTAAGGAGCACTTTCTGCTGAG-3' | 15 |
| **Isoform 2** | For 5'- GCGTTCCCCTCTGCTGAG | 7 |
| | Rev 5'-ACTCATTGCTGCAGGTAGATGAG | 8 |
| | Probe: FAM-TAMRA 5'-TTCTGGATGACTGCACTGTTCACTGGAGCT | 9 |
| **RPL37a** | For 5'-ACAGCGGAAGTGGTATTGTACGT | 10 |
| | Rev 5'-GGCACTGTGGTTCCTGCAT | 11 |
| | Probe: VIC-TAMRA 5'-CAGGCACCGCCAGCCACTGTCT | 12 |
| **PCR cloning of Isoform 2 cDNA** | For 5'-GGAGGAGGATGCGCATTTAAC-3 | 14 |
| | Rev 5'-TCTAAGGAGCACTTTCTGCTGAG-3' | 15 |
| **Isoform 3** | For 5'- AGCTTTTACCAGAATTCAGGTAGACTGT | 16 |
| | Rev 5'- TGCTGCAGGTAGATGAGGTCCT | 17 |
| | Probe: FAM-MGBNFQ 5'- CTTCTCCATCACTGATTTCATT | 18 |

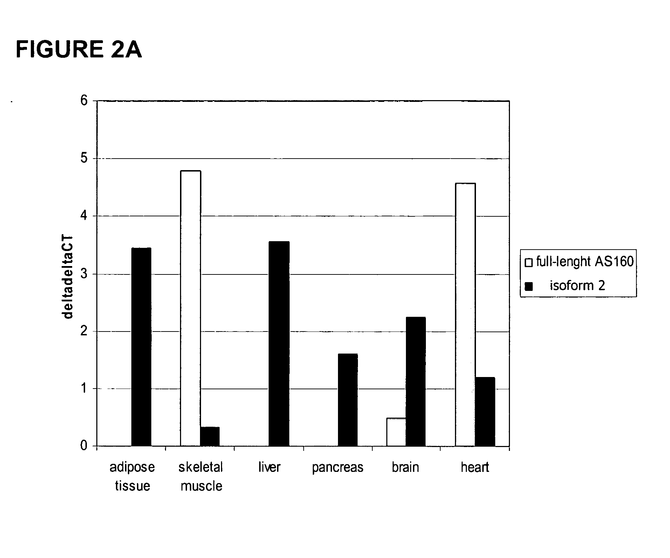

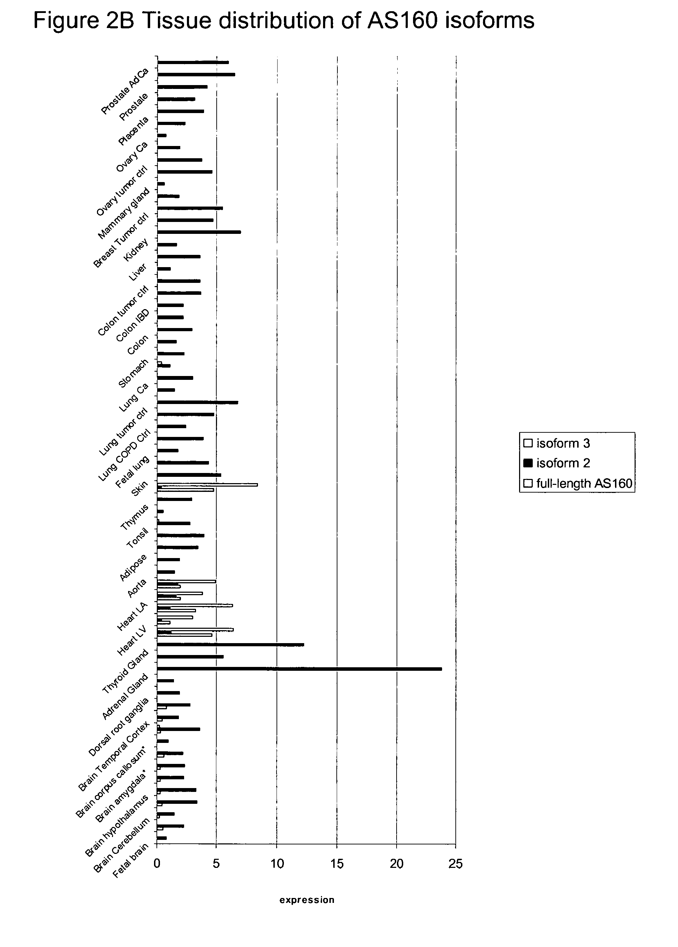

The results of this RT-PCR are shown in Figure 2. AS 160-protein (Isoform 1, MM_014832_v1; NCBI) represents full-length AS160. AS160-protein (Isoform 1) is mainly expressed in heart and skeletal muscle, but also in Skin (Figure 2A and B).

### Example 2: Cloning of AS160-expression construct and development of tetracycline-inducible expression in rat skeletal muscle cells

AS160-insert (Sequence ID NO:3) was amplified from human primary skeletal muscle cells, purchased by Cambrex/Lonza, using the primers 14 and 15. The obtained PCR fragment was used as a template for generating a fragment corresponding to the coding sequence of AS160 (isoforms 1). With corresponding gateway sequences the insert was first cloned into pDONR221 (Invitrogen) and subsequently into the expression vector pCDNA5-TO (Invitrogen) by means of standard methods.

L6 cells stably expressing GLUT4myc (L6-GLUT4myc, described in Wang *et al*. 1998) were subsequently used for the tet-inducible expression of AS160 protein. For this purpose the T-REx system from Invitrogen was used. The regulatory plasmid in this system controls the constitutive expression of the tet-repressor (tet-R) under the control of a CMV (cytomegalovirus) promoter. In the absence of tetracyline (or doxycycline) the repressor binds to specific tetracycline-operator sequences (TetO2) and thereby represses expression. Addition of tetracycline (or doxycycline) induces expression of the protein of interest. To allow a stable integration of the tet-system in L6-GLUT4myc cells the tet-repressor was islolated from pCDNA3.1 (+)ITR (Invitrogen) and cloned into the *NheI* and *NotI* sites of pIRESpuro2 (figure 3).

Subsequently pIRESpuro2/TR was transfected into L6-GLUT4myc cells. Clones stably expressing the regulatory plasmid were selected with 0.5 µg/ml puromycin (InvivoGen). Functionality of the tet-repressor was controlled with a tetracycline-inducible GFP expression plasmid (pCDNA5/TO-GFP). In the absence of tetracycline (or doxycyclin) only a small number of cells express GFP (Figure 3 A). Addition of doxycyclin increases the number of GFP expressing cells about 10 fold (Figure 3 B).

The obtained L6-GLUT4myc-tetR cells were deposited under the accession number DSM ACC2852 at the DSMZ (see above).

To obtain a tet-inducible expression of the AS160 protein, the above-generated pCDNA5-TO vector from Invitrogen containing the coding sequence of AS160 (isoform 1) was used. Selection of stable clones was performed with hygromycin (200 µg/ml, Invitrogen). Expression of AS160 was examined via western blot analysis with an AS160 specific antibody recognizing AS160 (Upstate, 07-741). Functionality of the tet-repressor was investigated with addition of doxycyclin (1µg/ml, Sigma) and subsequent western blot analysis.

The obtained L6-GLUT4myc-tetR-AS160_FL cells were deposited under the accession number DSM ACC2942 at the DSMZ (see above).

For all examples the L6-GLUT4myc cells containing AS160 were grown in MEMα (PAN) supplemented with 10% fetal calf serum (FCS) (PAA, tet-free), 2µg/ml blasticidin (Calbiochem), 0.5µg/ml puromycin (InvivoGen), 200µg/ml hygromycin (Invitrogen). Expression of of AS160 was induced with 1µg/ml doxycyclin (Sigma). L6-wildtype (wt) (ATCC: CRL-1458) cells were grown in MEMα + GlutaMax (Gibco) supplemented with 10% FCS (PAA, tet-free) and 1% penicillin/streptomycin (PAA). L6-GLUT4myc cells were grown in MEMα + GlutaMax (Gibco) supplemented with 10% FCS (PAA, tet-free), 1% penicillin/streptomycin (PAA) and 2 µg/ml blasticidin (Calbiochem). Expression of AS160 was induced by inbubation of the cells for 48 hours with 1 µg/ml doxycycline (Sigma). All cells were grown at 37°C and 5% CO₂. L6-GLUT4myc cells containing AS160 were incubated in starve medium (MEMα) 3-4 hours prior to each experiment.

For Western blot analysis proteins were separated on SDS-PAGE gels (4-12% resolving gel, Invitrogen), transferred to PVDF membranes (Roche) and blocked with Roti-Block® (Roth) for 1 hour. Membranes were incubated with primary antibodies overnight. The anti-AS160 antibody was from Upstate. Membranes were washed in TBST and incubated with the appropriate secondary horseradish peroxidase conjugated antibody (Santa Cruz). Immunoreactive bands were visualized with LumiLight (Roche) and detected with Lumi-Imager (Böhringer Ingelheim).

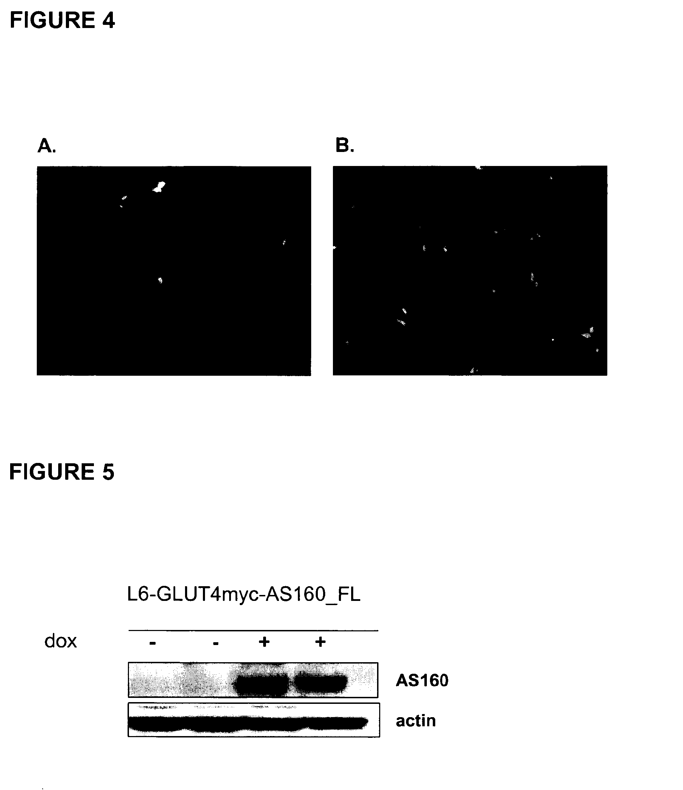

Figure 5 shows a representative western blot of doxycyclin-inducible expression of AS160- protein in L6-GLUT4myc cells containing the AS160- transcript.

### Example 3: Analysis of AS160 (isoform 1) using the above-generated L6-GLUT4myc cells containing AS 160 (isoform1)

In order to investigate the tetracycline-dependent expression of AS160, the cells described in example 2 were used for a western blot analysis. L6-GLUT4myc cells containing AS160 were grown in MEMα (PAN) supplemented with 10% fetal calf serum (FCS) (PAA, tet-free), 2µg/ml blasticidin (Calbiochem), 0.5µg/ml puromycin (InvivoGen), 200µg/ml hygromycin (Invitrogen. The expression of AS160 was induced with 1 µg/ml doxycycline for 48 hours as described above. Subsequently, cell extracts were prepared, separated via SDS-PAGE (4-12% resolving gel, Invitrogen) and transferred on a PVDF membrane (Roche) as described above. Membranes were blocked with Roti-Block® (Roth) for 1 hour. Incubation of the primary antibody was performed overnight. Membranes were washed in TBST. The appropriate secondary horseradish peroxidase conjugated antibody (Santa Cruz) was incubated for 2 hour at room temperature. Expression of AS160 was detected with an AS160 specific antibody (Upstate, 07-741). Equal loading was confirmed with an actin antibody (Santa Gruz, sc-1616) (Figure 5).

Immunoreactive bands were visualized with LumiLight (Roche) and detected with Lumi-Imager (Böhringer Ingelheim).

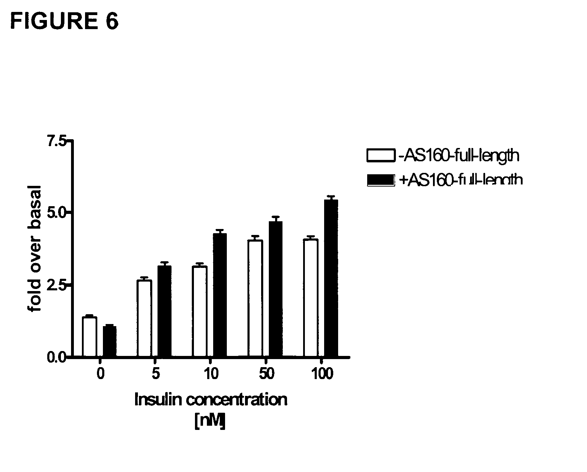

To examine glucose uptake of the cells, the respective cells were plated in 96 well Cytostar-T scintillating microplates (Amersham/GE). After 48 hours cells were serum-starved (3-4 hours) and treated with the indicated Insuman (human insulin) concentartionsa for 20 minutes. Uptake of 2-deoxyglucose (.01 mBq per well, Amersham/GE) was performed as already described (Voss et al. 2005). Nonspecific uptake was determined in the presence of 40 µM cytochalasin B (Calbiochem). This value was subtracted from all other values. Measurement occurred in a Wallac Microbeta counter (Perkin Elmer). Uptake of 2-deoxyglucose is measured as counts per million and presented as fold over basal. The data show that expression of AS160 (isoforms 1) increases the uptake of glucose up to about 6 fold after stimulation with insulin (concentration 100 nM insulin). Without expression of AS160 (isoforms 1) insulin induced glucose uptake up to a maximum of about 4 fold (Figure 6).

## Claims

1. A method of identifying a substance altering glucose uptake of a cell comprising
(a) contacting a test system comprising AKT substrate 160kDa- protein (AS160-protein) or functional variant thereof with a test substance, and
(b) identifying a test substance as a substance altering glucose uptake and/or GLUT4 translocation to the plasma membrane of a cell by detecting a signal indicative for altered glucose uptake of a cell.

2. The method of claim 1, wherein glucose uptake of the cell is increased or decreased, preferably increased.

3. The method of claim 1 or 2, wherein the substance alters glucose uptake and/or GLUT4 translocation to the plasma membrane in at least one, two or three insulin-sensitive tissues.

4. The method of claim 3, wherein the insulin-sensitive tissue is adipose tissue, skeletal muscle and/or liver.

5. The method of any of claims 1 to 4, wherein the substance alters glucose uptake and/or GLUT4 translocation to the plasma membrane of a hepatocyte, an adipocyte and/or a skeletal muscle cell.

6. The method of any of claims 1 to 5, wherein the AS160- protein is mammalian and preferably human AS160- protein.

7. The method of any of claims 1 to 6, wherein the AS160- protein comprises or consists of the sequence encoded by SEQ ID NO: 1 or xx.

8. The method of any of claims 1 to 7, wherein the test system is in a cell.

9. The method of claim 8, wherein the cell is a skeletal muscle cell, adipocyte and/or hepatocyte, particularly a skeletal muscle cell cell.

10. The method of claim 9, wherein the cell is a cell from a cell line.

11. The method of one of the claims 8 to 10, wherein the cell is a rodent or a mammalian cell, preferably a human or a murine or rat cell.

12. The method of any of claims 1 to 11, wherein the detectable signal is one or more of the following: the amount of AS 160- protein expressed in a cell, phosphorylated AKT, phosphorylated AS160- protein, GLUT4 translocation to the plasma membrane, GLUT4 distribution in a cell or glucose uptake by a cell.

13. The method of any of claims 1 to 12, wherein the test compound is provided in the form of a chemical compound library.

14. The method of any of the claims 1 to 13, wherein the method is carried out in a robotics system.

15. The method according to any of the claims 1 to 14, wherein the method is a method of high-through put screening.

16. A test system for the identification of a substance for improving glucose uptake and/or GLUT4 translocation to the plasma membrane of a cell, the test system comprising a gene coding for the AKT substrate 160 kDa protein (AS160-protein) or functional variant thereof; and an inducible promoter providing for controllable expression of the gene,
wherein the activation of AS160- protein or functional variant thereof effects a detectable signal.

17. The test system of claim 16, wherein the test system is located in a cell, particularly a genetically engineered cell.

18. The test system of claim 17, wherein the gene and/or the promoter is/are introduced into the genetically engineered cell.

19. The test system of any of claims 16 to 18, wherein the inducible promoter is a tetracycline-inducible promoter.

20. The test system of any of claims 16 to 19, wherein the test system is further defined as in any of claims 3 to 11.

21. Use of a tests system comprising AS160- protein for the identification of a substance altering, particularly improving, glucose uptake and/or GLUT4 translocation to the plasma membrane of a cell.

22. The use of claim 21, wherein the test system is further defined as in any claims 3 to 13 and 14 to 21.

23. Use of AS160- protein or a functional variant thereof in a model for type 2 diabetes.

24. Use of a cell heterotogously expressing AS 160- protein or a functional variant thereof as or in a model for type 2 diabetes.

25. The use of claims 23 or 24, wherein use involves a test system defined as in any claims 3 to 13 and 14 to 21.

26. Use of an inhibitor of AS160-protein, such as a siRNA or shRNA able to negatively interfere with expression and/or activity of AS160-like protein or an antibody able to negatively interfere with activity of AS 160-like protein for the inhibition or lowering of the Glucose uptake of a cell *in vitro* or *in vivo..*
